(19) Europäisches Patentamt
European Patent Office
Office européen des brevets

(11) **EP 3 777 974 B1**

(12) **EUROPÄISCHE PATENTSCHRIFT**

(45) Veröffentlichungstag und Bekanntmachung des Hinweises auf die Patenterteilung:
**05.07.2023   Patentblatt 2023/27**

(21) Anmeldenummer: **20190820.9**

(22) Anmeldetag: **13.08.2020**

(51) Internationale Patentklassifikation (IPC):
**A61N 5/06** (2006.01)

(52) Gemeinsame Patentklassifikation (CPC):
**A61N 5/062; A61N 5/0601;** A61B 2018/00005;
A61B 2018/00095; A61B 2018/00101;
A61B 2018/00148; A61B 2018/1807;
A61B 2018/2005; A61N 2005/005;
A61N 2005/0612; A61N 2005/0651

(54) **LICHTAPPLIKATOR**

LIGHT APPLICATOR

APPLICATEUR DE LUMIÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.08.2019   DE 102019212201**

(43) Veröffentlichungstag der Anmeldung:
**17.02.2021   Patentblatt 2021/07**

(73) Patentinhaber: **Richard Wolf GmbH**
**75438 Knittlingen (DE)**

(72) Erfinder:
• **Weber, Bernd Claus**
**76228 Karlsruhe (DE)**
• **Weigel, Manfred**
**75433 Maulbronn (DE)**
• **Sieber, Stephan**
**75438 Knittlingen-Freudenstein (DE)**
• **Müller, Klaus**
**75438 Knittlingen (DE)**
• **Wirth, Manfred**
**01326 Dresden (DE)**

(74) Vertreter: **Patentanwälte Vollmann Hemmer Lindfeld**
**Partnerschaft mbB**
**Wallstraße 33a**
**23560 Lübeck (DE)**

(56) Entgegenhaltungen:
**US-A1- 2014 188 035     US-A1- 2016 151 639**
**US-A1- 2016 213 945**

**Beschreibung**

Die vorliegende Offenbarung betrifft einen Lichtapplikator zur Untersuchung und/oder Behandlung eines organischen Körpers, insbesondere zur photodynamischen Therapie (PDT) von pathologischem Gewebe.

Es ist bekannt, Endoskope dazu zu nutzen, um Videoaufnahmen vom Inneren eines menschlichen oder tierischen Körpers zu Zwecken der medizinischen Diagnose und/oder Therapie zu machen. Dabei ist es ein ständiges Bestreben, den Einführabschnitt von Endoskopen möglichst dünn auszugestalten, damit möglichst kleine Hohlräume eingesehen werden können und das Gewebe dabei möglichst wenig in Mitleidenschaft zu ziehen.

Endoskope werden allerdings nicht nur dazu genutzt, um Bild- oder Videoaufnahmen zu machen, sondern auch als Diagnose- oder Therapiemittel selbst eingesetzt. Beispielsweise für die Detektion und Lokalisierung von prä- und frühmalignem Gewebe kann eine Fluoreszenz-Endoskopie eingesetzt werden, bei der es nicht auf eine natürliche Echtfarb-Darstellung des Gewebes ankommt, sondern lediglich auf eine Fluoreszenzanregung, mit der sich pathologisches Gewebe von gesundem Gewebe unterscheiden lässt. Dabei kann das mittels Lichtstrahlung angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise im Rahmen einer photodynamischen Diagnose (PDD) und/oder photodynamischen Therapie (PDT) mittels eines Photosensibilisators bzw. Markerstoffs (z.B. Chlorin e6) durchgeführt werden, der sich selektiv an pathologischem Gewebe anreichert.

Bei der photodynamischen Therapie (PDT) wird Licht mittels eines Lichtapplikators unmittelbar auf oder sogar in pathologisches Gewebe appliziert, um lichtinduziert die Bildung von Sauerstoffradikalen mittels des örtlich begrenzt angereicherten Photosensibilisators bzw. Markerstoffs zu fördern und dadurch das pathologische Gewebe, wie etwa einen Tumor, zu zerstören. Typischerweise wird dazu Laserlicht in einen Lichtleiter eingekoppelt und zum Gewebe geleitet. Wenn das pathologische Gewebe flächig auf einer äußeren Oberfläche, z. B. die Haut, oder einer inneren Oberfläche, z. B. Speiseröhreninnenfläche oder Darmwandung, angeordnet ist, dann kann das Therapielicht relativ einfach ausgekoppelt und auf die pathologische Gewebefläche gestrahlt werden. Erstreckt sich allerdings das pathologische Gewebe über ein Volumen, so kann wegen der begrenzten Eindringtiefe des Lichts in das Gewebe nicht immer ein Tumor von "außen" effektiv bestrahlt werden. In diesem Fall ist die PDT besonders effektiv, wenn das Licht vom Inneren des pathologischen Gewebevolumens aus möglichst isotrop abgestrahlt wird. Dazu muss der Lichtapplikator in das pathologische Gewebe eingestochen werden. Dies wird auch als interstitielle (durch innere Oberflächen) und/oder perkutane (durch die Haut) PDT bezeichnet.

In der EP 2 449 994 A1 ist beispielsweise beschrieben, wie ein Lichtapplikator in Form eines Laserlichtleiters in pathologisches Gewebe eingeschoben wird, nachdem der Weg für den Lichtleiter durch das Gewebe mit einer Nadel vorgestochen wurde. Die US 2016/151639 A1 beschreibt ein medizinisch-endoskopisches Gerät zum Einführen in einen Körper und Bestrahlung mit Licht. Die US 2016/213945 A1 und die US 2014/0188035 A1 beschreiben Kathederlösungen für die PDT.

Nachteilig an dieser bekannten Lösung ist einerseits, dass die Lichtauskopplung aus einem dünnen distalen Laserlichtleiterende nicht isotrop, sondern stark distalwärts gebündelt ist. Andererseits ist der dünne Laserlichtleiter selbst sehr biegsam, wodurch er mittels einer starren Kanalführung möglichst weit bis zum pathologischen Gewebe geführt sein muss und nicht weit aus der Kanalführung geschoben werden kann, ohne dass er sich verbiegt. Es müssen beim Einstechen je nach Eindringtiefe gegebenenfalls große Widerstände von zu durchdringendem gesundem und pathologischem Gewebe überwunden werden, sodass die mögliche Eindringtiefe bei der bekannten Lösung sehr begrenzt ist. Außerdem ist die bekannte Lösung relativ komplex und teuer, sodass sie nicht als Einweg-Artikel zum einmaligen Gebrauch realisierbar ist.

Daraus ergibt sich die Aufgabe, einen kostengünstigeren Lichtapplikator bereitzustellen, der eine effektivere Raumausleuchtung und eine höhere Eindringtiefe für interstitielle und/oder perkutane PDT erlaubt.

Gemäß der vorliegenden Offenbarung wird zur Lösung dieses Problems ein Lichtapplikator zur Untersuchung und/oder Behandlung von einem organischen Körper bereitgestellt, wobei der Lichtapplikator einen distalseitigen Nadelabschnitt mit einer LED am distalen Ende zum Einstechen in Gewebe des organischen Körpers und einen proximalseitigen Kabelabschnitt aufweist. Der Lichtapplikator weist dabei einen elektrischen Leiter zur Stromversorgung der LED auf, wobei der Leiter im Nadelabschnitt massiv und in einer Weise dicker als im Kabelabschnitt ausgeführt ist, dass die Querschnittsfläche des Leiters im Nadelabschnitt mehr als 70% der Querschnittsfläche des Lichtapplikators beträgt und somit der Nadelabschnitt biegesteifer als der Kabelabschnitt ist. Mit Biegesteifheit sei in dieser Offenbarung auch eine Torsionssteifheit gemeint. Das heißt, dass sich der Nadelabschnitt nicht nur weniger verbiegt, sondern auch weniger verdreht als der Kabelabschnitt.

Bei dem hier offenbarten Lichtapplikator wird also kein Laserlichtleiter eingesetzt, sondern das Therapielicht in situ am distalen Ende des Lichtapplikators durch eine miniaturisierte LED erzeugt, z. B. mit einer lateralen Breite von weniger als 1 mm. Ein teurer Laser wird daher nicht benötigt. Die gewöhnliche Abstrahlcharakteristik einer LED gemäß einem Lambert'schen Strahler deckt bereits ohne Hilfe einer optischen Komponente einen weitaus größeren Raumwinkel ab als eine Laserlichtauskopplung aus einem Laserlichtleiter. Wird vorzugsweise die LED mit einer optischen Komponente in Form eines Streukörpers distalseitig ergänzt, so kann ein sehr großer Raumwinkel mit relativ homogen verteilter Leistung bestrahlt werden, um dem Ideal einer isotropen Raumausleuchtung möglichst nah zu kommen.

Der hierin offenbarte Lichtapplikator nutzt den für die Stromversorgung der LED notwendigen elektrischen Leiter dazu, den distalen Endabschnitt des Lichtapplikators zu einem Nadelabschnitt zu versteifen, indem er so dick ausgebildet ist, dass er einen Großteil der Querschnittsfläche des Lichtapplikators ausmacht. Der distale Endabschnitt des Lichtapplikators ist damit selbst eine relativ biegesteife Nadel mit im Vergleich zum Laserlichtleiter weitaus größerer möglicher Eindringtiefe, ohne dass er sich beim Einstechen unter den Widerstand des Gewebes verbiegt. Prinzipiell bringt die In-Situ-Lichterzeugung mit einer LED zwar den Nachteil mit sich, dass die LED Wärme erzeugt, die das Gewebe möglichst nicht in schädigender Weise erhitzen darf. Allerdings fungiert der relativ dicke elektrische Leiter nicht nur als Versteifungselement und Versorgungselement mit elektrischer Energie, sondern auch als sehr guter Wärmeleiter in proximale Richtung. Vorzugsweise kann mit einer lateral umgebenden wärmeisolierenden Schicht bzw. Mantel, z. B. aus Kunststoff, die Wärme proximalwärts über den elektrischen Leiter abgeführt werden, bevor Gewebe in schädigender Weise erhitzt wird. Der elektrische Leiter kann dazu vorzugsweise proximalseitig gekühlt und/oder thermisch mit einer Wärmesenke gekoppelt sein.

Der hierin offenbarte Lichtapplikator kann sehr günstig hergestellt werden und somit als steriler Einweg-Artikel zum einmaligen Gebrauch realisiert werden, was aufwendige Reinigungen und Sterilisationen beim Anwender obsolet macht. Bei größeren Tumoren oder ganzen pathologischen Organen oder Organbereichen kann eine Mehrzahl an hierin offenbarten Lichtapplikatoren gleichzeitig für die PDT eingesetzt werden, indem sie verteilt über das gesamte Organ eingestochen werden, um das gesamte Organ homogen auszuleuchten. Da sich der Photosensibilisator bzw. Markerstoff (z.B. Chlorin e6) selektiv nur an pathologischem Gewebe anreichert und dort unter dem Lichteinfluss reagiert, wird gesundes Gewebe durch das Licht nicht beschädigt. Zum einen muss das pathologische Gewebe dann zuvor nicht mehr so genau lokalisiert werden und zum anderen wird das Risiko verringert, dass pathologisches Gewebe unbemerkt untherapiert bleibt. Für die perkutane PDT mit mehreren Lichtapplikatoren kann es sinnvoll sein, eine flächig vor oder auf die Haut des Patienten platzierbare und/oder klebbare, ggf. organspezifische Schablone bzw. Template, mit organspezifischen Markierungen und/oder Durchbrechungen bereitzustellen, um einem Anwender damit Einstichstellen, -winkel und/oder -tiefen für die Lichtapplikatoren anzuzeigen und eine möglichst vollständige Ausleuchtung eines Organs zu erzielen.

Der Lichtapplikator kann allerdings nicht nur für die Behandlung, also Therapie eingesetzt werden, sondern auch für die Untersuchung, also die Diagnose. Insbesondere im Zusammenspiel mit einem Endoskop kann die durch den Lichtapplikator erzeugte Fluoreszenz eines an pathologischem Gewebe angereicherten Photosensibilisators bzw. Markerstoffs (z.B. Chlorin e6) beobachtet werden.

Der Lichtapplikator kann durch einen Arbeitskanal eines endoskopischen Instruments geschoben werden und am distalen Ende mit seinem Nadelabschnitt in das Gewebe gestochen werden, was vorzugsweise mit einem distalseitigen Bildsensor am endoskopischen Instrument beobachtbar ist. Dies ist insbesondere für die interstitielle PDT sinnvoll, wenn beispielsweise durch natürliche Körperöffnungen, wie etwa Darm, Harnleiter, Speise- oder Luftröhre der Weg zum Tumor mittels eines endoskopischen Instruments verkürzt werden kann. Der Lichtapplikator kann allerdings auch ganz ohne endoskopisches Instrument beispielsweise für die perkutane PDT verwendet werden, bei der der Nadelabschnitt beispielsweise CT- oder ultraschallunterstützt von außen durch die Haut bis in das pathologische Gewebe gestochen wird. Der Nadelabschnitt kann vorzugsweise eine oder mehrere abstrakte oder konkrete laterale Längenmarkierungen aufweisen, die dem Anwender beim Einstechen eine relative oder absolute Einstichtiefe vermitteln.

Optional ist die LED an einer distalen Stirnseite des Leiters angeordnet, sodass die Hauptabstrahlrichtung der LED distalwärts in Längsrichtung des Lichtapplikators verläuft. Die LED steht vorzugsweise sowohl in elektrisch leitendem als auch in wärmeleitendem Kontakt mit der distalen Stirnseite des Leiters.

Optional können der Nadelabschnitt und der Kabelabschnitt lösbar miteinander verbindbar oder fest miteinander verbunden sein. Es kann für die modulare Produktion vorteilhaft sein, wenn der Nadelabschnitt und der Kabelabschnitt lösbar miteinander verbindbar sind, wenn der Nadelabschnitt je nach Anwendungsfall anderen Anforderungen genügen muss, wohingegen der Kabelabschnitt für mehrere Anwendungsfälle gleich ausgestaltet sein kann. Außerdem kann ein Nadelabschnitt, von dem der Kabelabschnitt getrennt oder noch nicht angeschlossen wurde, die Handhabung des Nadelabschnitts beim Vorgang des Platzierens des Nadelabschnitts im Gewebe vereinfachen. Eine feste Verbindung kann von Vorteil sein, wenn beispielsweise im Kabelabschnitt Identifikationsmittel, wie etwa eine mechanische oder digitale Steckerkennung, vorgesehen sind, die eine automatische Identifikation des Lichtapplikators ermöglichen.

Optional kann der Leiter einen Kern mit einem ersten Material und einen Mantel mit einem zweiten Material aufweisen, wobei das erste Material wärmeleitfähiger ist als das zweite Material und das zweite Material biegesteifer ist als das erste Material. Das erste Material kann beispielsweise Kupfer, Aluminium oder Silber und das zweite Material Stahl oder eine andere Legierung mit einem vergleichsweise hohen Elastizitätsmodul aufweisen. Bei dieser bevorzugten Ausführungsform wird mit dem vergleichsweise harten Mantelmaterial, z.B. Stahl, bei möglichst geringem Materialaufwand eine hohe Biegesteifigkeit für den Nadelabschnitt erzielt. Der Kern aus wärmeleitfähigerem Material, z.B. Kupfer, Aluminium oder Silber, kann damit einen möglichst großen Querschnittsanteil des Leiters ausmachen, ohne die Biegesteifigkeit signifikant zu beeinträchtigen. Damit wird ein guter Kompromiss zwischen Biegesteifigkeit und proximalwärtiger Wärmeleitfähigkeit erzielt. Vorzugsweise beträgt die Wärmeleitfähigkeit des Kerns mindestens das Vierfache der Wärmeleitfähigkeit des Mantels. Die Wärmeleitfähigkeit von Kupfer und Silber als Kern kann beispielsweise mehr als 400 W/(Km) bei 20°C betragen, diejenige von Aluminium mehr als 200 W/(Km), wogegen die Wärmeleitfähigkeit von Stahl als Mantel deutlich unter 100 W/(Km), üblicherweise im Bereich von 50 W/(Km), bei 20°C liegt.

Optional kann die Querschnittsfläche des Kerns mehr als 40% der Querschnittsfläche des Leiters betragen. Vorzugsweise hat der Kern einen möglichst großen Querschnittsanteil am Leiter, um möglichst viel Wärme von der LED proximalwärts abführen zu können. Der Querschnittsanteil des Mantels ist möglichst klein, allerdings groß genug, um eine ausreichende Biegesteifigkeit zu erzielen. Der Mantel kann beispielsweise eine Dicke von etwa 50 $\mu$m bis 300 $\mu$m haben, während der Kern einen Durchmesser von etwa 1 mm haben kann.

Optional kann die Querschnittsfläche des Kerns das 0,8 bis 1,2-fache der Querschnittsfläche der LED betragen. Die Designfreiheit, den Lichtapplikator möglichst dünn und damit minimalinvasiv auszugestalten, kann durch die laterale Breite und Form der momentan am Markt verfügbaren bzw. herstellbaren kleinstmöglichen LEDs begrenzt sein. Es ist daher vorteilhaft, den Durchmesser des Leiters oder dessen Kerns im Wesentlichen der laterale Breite der LED anzupassen. Dann kann die LED über ihre gesamte Querschnittsfläche besonders effektiv proximalwärts Wärme an den Leiter oder dessen Kern abgeben, ohne den Lichtapplikator insgesamt unnötig verdicken zu müssen. Die Querschnittsform des Leiters und/oder dessen Kerns kann ebenfalls an die Querschnittsform der LED angepasst sein. Beispielsweise kann bei einer rechteckigen oder quadratischen Querschnittsform der LED auch der Leiter und/oder dessen Kern eine entsprechend rechteckige oder quadratische Querschnittsform aufweisen.

Optional kann die Querschnittsfläche des Leiters mindestens so groß wie die Querschnittsfläche der LED sein. Der dünne und biegesteife Mantel kann dabei die LED lateral einfassen, also ein Stück weit hülsenförmig distalwärts über den Kern hinausragen. Der Leiter kann beispielweise eine runde Querschnittsform aufweisen mit einem Durchmesser, der mindestens der Querschnittsdiagonalen einer rechteckigen oder quadratischen LED entspricht.

Optional kann der Kern mit dem ersten Material mit einer hohen Wärmeleitfähigkeit als Hinleiter und der Mantel mit dem zweiten Material mit einem hohen Elastizitätsmodul als Rückleiter genutzt werden oder umgekehrt. Dabei können Kern und Mantel durch eine dünne, elektrisch nichtleitende Schicht, die zwischen den beiden Komponenten angeordnet sein kann, gegeneinander elektrisch isoliert sein. Dies hat den Vorteil, dass im Bereich des Nadelabschnitts auf einen separat ausgeführten zweiten elektrischen Leiter, d.h. auf einen zweiten elektrischen Leiter, der als zusätzliche Komponente ausgeführt ist, verzichtet werden kann, was den Durchmesser des Nadelabschnitts als demjenigen Teil, welcher in den Körper eingeführt wird, in vorteilhafter Weise reduzieren kann.

Optional kann der Lichtapplikator im Nadelabschnitt in radiale Richtung derart wärmegedämmt sein, dass die radiale Wärmedämmung proximalwärts abnimmt. Damit wird ein besonders effizientes Wärmemanagement erzielt, das bei möglichst geringem Materialaufwand einen proximalwärtigen Wärmetransport erlaubt, ohne dabei das angrenzende Gewebe in schädigender Weise zu erhitzen. Technisch besteht nämlich die Herausforderung darin, dass die LED eine relativ große Wärmeleistung erbringt und die LED ohne ausreichenden Wärmeabtransport sehr schnell Schaden nimmt, oder zumindest die von ihr zu erbringende optische Leistung abnimmt. Würde man einerseits zum Schutz des umgebenden Gewebes einfach eine sehr starke radiale Wärmedämmung über die gesamte Länge des Nadelabschnitts vorsehen, würde die LED überhitzen und Schaden nehmen, oder zumindest die von ihr zu erbringende optische Leistung abnehmen. Würde man andererseits zum Schutz der LED einfach eine sehr schwache radiale Wärmedämmung über die gesamte Länge des Nadelabschnitts vorsehen, würde das umgebende Gewebe im Bereich der LED bzw. im Bereich der Nadelspitze zu stark erhitzt und thermisch bedingten Schaden nehmen. Dies würde auch gesundes Gewebe betreffen, wenn sich dieses genügend nahe der LED bzw. der Nadelspitze befindet, d.h. dass die die PDT in vorteilhafter Weise kennzeichnende Selektivität bei der Gewebezerstörung, wonach nur das pathologische Gewebe zerstört wird, bei solch einem Applikator verloren ginge. Außerdem können hohe Temperaturen am Gewebe entsprechend starke Schmerzen verursachen, denen ggf. mit einer Narkotisierung des Patienten begegnet werden müsste, was zusätzlichen Aufwand für den Anwender und zusätzliche Belastung für den Patienten bedeutete. Als Lösung dieses Problems wird mittels der proximalwärts abnehmenden radialen Wärmedämmung ein proximalwärtiger Wärmefluss erzielt, der zum einen die LED vor Überhitzung schützt und die laterale Wärmeabgabe an das umgebende Gewebe insoweit proximalwärts über den Nadelabschnitt verteilt, dass sich das umgebende Gewebe nur noch in vertretbarem Maße erhitzt.

Optional kann die radiale Wärmedämmung im Nadelabschnitt proximalwärts stufenweise und/oder stetig abnehmen. Bei einer stufenweisen proximalwärtigen Abnahme der Wärmedämmung weist der Nadelabschnitt vorzugsweise zwei oder mehr Stufen der Wärmedämmung auf, die distal vom proximalen Ende des Nadelabschnitts angeordnet sind. Die radiale Wärmedämmung, d.h. der thermische Widerstand $R_{th} = \frac{\Delta T}{\dot{Q}}$ in radiale Richtung, welcher die einen Wärmefluss $\dot{Q} = \frac{dQ}{dt}$ in radiale Richtung bewirkende Temperaturdifferenz dT bestimmt, ist durch die Dicke und die Wärmeleitfähigkeit des Materials der Wärmedämmung bestimmt. Die Wärmedämmung kann aus einer oder mehreren Wärmedämmschichten gebildet sein, wobei eine Mehrzahl an Wärmedämmschichten aus jeweils gleichen oder aus unterschiedlichen Materialien mit unterschiedlichen Wärmeleitfähigkeiten gebildet sein kann. Bei einem kreisförmigen Querschnitt des Nadelabschnitt kann der thermische Widerstand $R_{th} = \frac{\Delta T}{\dot{Q}}$ einer Wärmedämmschicht in radiale Richtung näherungsweise durch folgende Formel dargestellt werden:

$$R_{th} = \frac{\ln\left(\frac{r_a}{r_i}\right)}{2\pi\lambda L},$$

wobei $r_a$ der Außenradius der Wärmedämmschicht, $r_i$ der Innenradius der Wärmedämmschicht, L die Länge der Wärmedämmschicht in Längsrichtung des Nadelabschnitts und A die Wärmeleitfähigkeit der Wärmedämmschicht ist. Für eine Wärmedämmschicht aus PET kann die Wärmeleitfähigkeit beispielsweise bei unter 0,3 W/(Km) bei 20°C liegen. Bei einer Mehrzahl von Wärmedämmschichten können die einzelnen Beiträge $R_{th}$ jeder Wärmedämmschicht zur gesamten Wärmedämmung addiert werden.

Die radiale Wärmedämmung im Nadelabschnitt, d.h. der thermische Widerstand $R_{th} = \frac{\Delta T}{\dot{Q}}$ in radiale Richtung, an der LED ist vorzugsweise mindestens doppelt hoch wie um das 10-fache des Lichtapplikatordurchmessers an der LED in proximale Richtung entfernt von der LED. Um das 20-fache des Lichtapplikatordurchmessers an der LED in proximale Richtung entfernt von der LED kann die Wärmedämmung auf weniger als ein Drittel reduziert sein gegenüber der radialen Wärmedämmung an der LED. Wenn also der Lichtapplikatordurchmesser an der LED 1 mm beträgt, so wäre die radiale Wärmedämmung im Abstand von 1 cm von der LED in proximale Richtung nur noch maximal halb so stark wie an der LED. Im Abstand von 2 cm von der LED in proximale Richtung betrüge dann die radiale Wärmedämmung nur noch höchstens 33% der radialen Wärmedämmung an der LED.

Optional kann der Lichtapplikator im Nadelabschnitt mindestens eine radiale Wärmedämmschicht aufweisen, wobei die Gesamtdicke der mindestens einen Wärmedämmschicht proximalwärts abnimmt; Dabei kann die Gesamtdicke der mindestens einen Wärmedämmschicht proximalwärts abnehmen und/oder deren Anzahl. Zusätzlich oder alternativ dazu kann die Wärmeleitfähigkeit des Materials der Wärmedämmung proximalwärts zunehmen, d.h. es können in Längsrichtung des Nadelabschnitts unterschiedliche Wärmedämmmaterialien angeordnet sein.

Optional kann der Durchmesser des Leiters in dem Maße zunehmen wie die radiale Wärmedämmung proximalwärts abnimmt. Dies ist insbesondere dann vorteilhaft, wenn die Gesamtdicke der mindestens einen Wärmedämmschicht proximalwärts abnimmt und die Querschnittsfläche des Lichtapplikators über die Länge des Einführabschnitts im Wesentlichen konstant ist. Dort, wo die Wärmedämmung dünner ist, kann also der Leiter entsprechend dicker ausgestaltet sein, um den thermischen Widerstand des Leiters in proximale Richtung, der sich reziprok zur Querschnittsfläche des Leiters verhält, zu verringern.

Optional kann im Nadelabschnitt die Wärmeleitfähigkeit des Materials der radialen Wärmedämmung proximalwärts zunehmen. Es können also in Längsrichtung verteilt über den Nadelabschnitt unterschiedliche Materialien die Wärmedämmung bilden und/oder es erstrecken sich bestimmte Wärmedämmschichten nur über bestimmte Längenabschnitte des Nadelabschnitts.

Optional kann der Nadelabschnitt eine sich zumindest teilweise distalwärts von der LED angeordnete und sich distalwärts verjüngende Nadelspitze mit einem lichttransparenten Streukörper zur Streuung des Lichts der LED aufweisen. Vorzugsweise weist die Nadelspitze einen hülsenförmigen proximalen Abschnitt auf, der die LED und vorzugsweise einen distalen Teil des Leiters lateral umgibt und zumindest teilweise eine radiale Wärmedämmung an der LED bildet. Der lichttransparente Streukörper und der hülsenförmige proximale Abschnitt können beispielsweise im Wesentlichen einstückig aus Kunststoff, beispielsweise aus Epoxidharz, gebildet sein, wobei das Epoxidharz eine Wärmeleitfähigkeit von beispielsweise unter 2 W/(Km) bei 20°C haben kann. Der hülsenförmige proximale Abschnitt der Nadelspitze kann wiederum ganz oder teilweise von einer oder mehreren Wärmedämmschichten umgeben sein. Der hülsenförmige proximale Abschnitt der Nadelspitze kann zur sicheren Befestigung der Nadelspitze am Leiter des Nadelabschnitts dienen.

Optional kann eine zusätzliche Verbesserung der Befestigungssicherheit dadurch erreicht werden, dass der Leiter außenseitig mindestens eine Vertiefung oder Aufweitung aufweist, beispielweise als lokale Querschnittsverjüngung oder -vergrößerung. Dadurch wird erreicht, dass der hülsenförmige proximale Abschnitt der Nadelspitze mit dem Leiter einen Formschluss bilden kann. Die Nadelspitze kann als Kunststoffgussteil oder als Kunststoffspritzgussteil ausgebildet sein, das beispielsweise durch Umspritzen oder Umgießen mit der außenseitigen Vertiefung oder Aufweitung des Leiters einen Formschluss mittels eines Hinterschnitts bilden kann.

Optional kann das Volumen der Nadelspitze im Wesentlichen vom Streukörper ausgebildet sein und in einem spitzen und/oder kantenförmigen Nadelspitzenendbereich ein Verstärkungselement aufweisen. Dies ist besonders vorteilhaft, um eine möglichst scharfe und damit minimalinvasive lichttransparente Nadelspitze bereitzustellen, die sowohl sicher als auch kostengünstig ist. Der Werkstoff dieses Verstärkungselements kann vorzugsweise einerseits eine hohe Festigkeit und andererseits eine hohe Zähigkeit aufweisen. Die hohe Festigkeit ist vorteilhaft für scharfe und dementsprechend dünne, aber dennoch stark belastbare Kanten und/oder Spitzen. Die hohe Zähigkeit ist vorteilhaft, um bei potenzieller Überbelastung der scharfen Kanten und/oder Spitzen ein Abbrechen von Kanten-/Spitzenteilen zu vermeiden. Dies stellt insbesondere ein Problem bei Materialien mit hoher Sprödigkeit, wie etwa Glas, dar. Ein Werkstofftyp, der beide Eigenschaften, d.h. hohe Festigkeit und hohe Zähigkeit, vereinen kann, ist beispielsweise Metall. Würde ein sprödes Glas verwendet, wäre der Nadelspitzenendbereich bei Belastung nicht sicher gegen ein Abbrechen. Ein transparenter Kunststoff wie Epoxidharz ist nicht hart genug, also zu biegsam, um ihn im Nadelspitzenendbereich scharf genug auszubilden, ohne dass er sich bei Belastung im Nadelspitzenendbereich biegen würde. Durch das Verstärkungselement kann der Nadelspitzenendbereich so verstärkt werden, dass ein günstig herstellbarer transparenter Kunststoff wie Epoxidharz als Basismaterial für den Streukörper der Nadelspitze verwendet werden kann. Da ein solcher Kunststoff relativ einfach gegossen, spritzgegossen, oder in vergleichbarer anderer Form verarbeitet werden kann, ist es im Gegensatz zu Glas, Kristall, usw. auch möglich, den oben beschriebenen Formschluss zur Verbesserung der Befestigungssicherheit der Nadelspitze am Applikator zu erzielen, indem die Nadelspitze beispielsweise durch Umspritzen oder Umgießen der LED und eines distalen Abschnitts des Leiters geformt wird.

Optional kann der Streukörper im Wesentlichen aus lichtstreuendem Kunststoff und/oder lichttransparentem Kunststoff mit lichtstreuenden Partikeln und das Verstärkungselement aus Metall, beispielsweise Stahl, ausgebildet sein. Der Anteil der Querschnittsfläche der Nadelspitze, der vom nicht lichttransparentem Verstärkungselement eingenommen wird, kann vernachlässigbar klein sein, z. B. unter 15% liegen.

Optional kann das Verstärkungselement als ein in den Streukörper zumindest teilweise eingebetteter Dorn oder als in den Streukörper zumindest teilweise eingebettete Klinge ausgeführt sein. Die jeweils bevorzugte Ausgestaltung kann von der Anwendung abhängen. Bei einer perkutanen PDT an einem Prostatakarzinom kann es beispielsweise sein, dass der Lichtapplikator auf dem Weg zum Prostatakarzinom gesunde Nervenbahnen oder andere sensible Gefäße passiert, die nicht verletzt oder durchtrennt werden sollen. Eine dornförmige Ausführung des Verstärkungselements kann dann das Risiko einer ungewollten Verletzung von gesundem Gewebe, Nerven oder Gefäßen signifikant verringern. Bei einer perkutanen PDT an einem Mammakarzinom hingegen könnte die Verletzung oder Durchtrennung von gesundem Fettgewebe weniger problematisch sein und eine klingenförmige Ausführung des Verstärkungselements den Widerstand beim Einstechen verringern, die Narbenbildung reduzieren und den Heilungsprozess verbessern.

Optional kann das Verstärkungselement zumindest teilweise distalwärts und/oder lateral aus dem Streukörper herausragen. Dann wirkt das Verstärkungselement hauptsächlich als Einstichnadel oder -klinge und weniger der Nadelspitzenendbereich des Streukörpers selbst, in den es zumindest teilweise eingebettet ist. Das metallische Verstärkungselement kann dann distalseitig sehr spitz und/oder scharf wie ein Skalpell ausgebildet sein.

Optional kann das Verstärkungselement in der radialen Mitte des Streukörpers weiter distalwärts aus dem Streukörper herausragen als am radialen Außenbereich des Streukörpers. Damit kann insbesondere bei einer klingenförmigen Ausführungsform eine zentrale Spitze erreicht werden, von der sich Klingenabschnitte schräg in radial-proximale Richtung erstrecken, um beim Einstechen des Lichtapplikators einen guten Schneideffekt ohne lateralen Abdrift zu erzielen. Des Weiteren reduziert eine solche Maßnahme die vom Anwender aufzubringende Kraft beim Einführen und Vorschieben des Applikators in das Gewebe.

Optional kann das Verstärkungselement lateralseitig eine spiegelnde Oberfläche aufweisen. Dadurch wird die ohnehin geringe Beeinträchtigung der Lichtabstrahlung durch das nicht lichttransparente Verstärkungselement weiter verringert.

Optional kann der Streukörper in einer ersten Längsschnittebene in einem ersten Winkel distalwärts zulaufen und in einer zur ersten Längsschnittebene senkrecht liegenden zweiten Längsschnittebene in einem zweiten Winkel distalwärts zulaufen, wobei der zweite Winkel spitzer ist als der erste Winkel, In der ersten Längsschnittebene kann beispielsweise die Haut und das Gewebe geschnitten werden, während die Einstichöffnung in der zweiten Längsschnittebene vom Streukörper lediglich aufgeweitet wird. Dies wird durch stumpfe Kantenwinkel in der zweiten Längsschnittebene begünstigt. Dies kann den Heilungsprozess verbessern und die Narbenbildung reduzieren. Optional kann dabei das Verstärkungselement in der ersten Längsschnittebene angeordnet sein und den darin verlaufenden kantenförmigen Nadelspitzenendbereich verstärken. Ist die Kante des Verstärkungselements entsprechend scharf ausgeführt, begünstigt dies zusätzlich die Schneidwirkung in der ersten Längsschnittebene.

Optional kann der Streukörper polyedrisch sein mit einem ersten Streukörperabschnitt, einem distal vom ersten Streukörperabschnitt angeordneten zweiten Streukörperabschnitt und einem distal vom zweiten Streukörperabschnitt angeordneten dritten Streukörperabschnitt, wobei der erste Streukörperabschnitt einen im Wesentlichen quadratischen Querschnitt hat, wobei der zweite Streukörperabschnitt einen im Wesentlichen achteckigen Querschnitt hat, und wobei der dritte Streukörperabschnitt einen im Wesentlichen rautenförmigen Querschnitt hat. Beim Einstich des distalseitigen dritten Streukörperabschnitts weitet sich die im Wesentlichen rautenförmige Einstichöffnung in Richtung beider Rautendiagonalen auf, wobei vorzugsweise das Verstärkungselement die Einstichöffnung entlang der längeren Rautendiagonalen aufschneidet. Sobald der zweite mittlere Streukörperabschnitt die Einstichöffnung erreicht, ist die maximale Öffnung entlang der längeren Rautendiagonalen bereits erreicht und die Einstichöffnung wird mit achteckigem Querschnitt senkrecht zur Schnittebene des Verstärkungselemente geweitet. Da vorzugsweise alle Winkel derjenigen Kanten, die nicht das Verstärkungselement enthalten, stumpf sind, findet in der Richtung senkrecht zur Ebene mit dem Verstärkungselement im Wesentlichen nur noch eine Dehnung/Weitung des Gewebes und kein Schnitt statt. Der proximale erste Streukörperabschnitt hat dann den im Wesentlichen quadratischen Querschnitt des Nadelabschnitts, sodass dann die Einstichöffnung die maximale Größe zum Einführen des Nadelabschnitts erreicht hat. Es hat sich gezeigt, dass die Heilung einer derart gebildeten Einstichöffnung mit weniger Narbenbildung einhergeht.

Optional kann das Verstärkungselement als zwei im Querschnitt gekreuzt zueinander angeordnete Klingen ausgebildet sein. Diese Ausführungsform kann für besonders festes Gewebe vorteilhaft sein, das weniger dehnbar ist und besser verheilt, wenn es gekreuzt geschnitten wird als wenn es gedehnt wird.

Die Begriffe "distalwärtig" bzw. "proximalwärtig" sollen hierin eine relative Position bedeuten, die sich distal bzw. proximal von einem Anwender des Systems als Bezugsposition befindet. Die Begriffe "distalseitig" bzw. "proximalseitig" sollen hierin entsprechend eine Position an einer distalen bzw. proximalen Seite eines Gegenstands bedeuten. Die Begriffe "distalwärts" bzw. "proximalwärts" soll hierin entsprechend eine Richtung bedeuten, die sich nach distal bzw. proximal erstreckt.

Die Offenbarung ist nachfolgend anhand von in den Zeichnungen dargestellten Ausführungsbeispielen näher erläutert. Es zeigen:

Fig. 1 eine schematische Darstellung einer beispielhaften Ausführungsform eines hierin offenbarten Lichtapplikatorsystems;

Fig. 2 eine schematische Darstellung einer anderen beispielhaften Ausführungsform eines hierin offenbarten Lichtapplikatorsystems;

Fig. 3 eine schematische Darstellung eines Beispiels eines Lichtapplikators einer beispielhaften Ausführungsform eines hierin offenbarten Lichtapplikatorsystems;

Fig. 4 eine schematische Darstellung eines anderen Beispiels eines Lichtapplikators einer beispielhaften Ausführungsform eines hierin offenbarten Lichtapplikatorsystems;

Fig. 5 eine schematische Darstellung einer weiteren beispielhaften Ausführungsform eines hierin offenbarten Lichtapplikatorsystems;

Fig. 6 eine schematische Darstellung einer weiteren beispielhaften Ausführungsform eines hierin offenbarten Lichtapplikators;

Fig. 7 eine schematische Darstellung einer weiteren beispielhaften Ausführungsform eines hierin offenbarten Lichtapplikators;

Fig. 8 eine schematische Darstellung einer weiteren beispielhaften Ausführungsform eines hierin offenbarten Lichtapplikators;

Fig. 9a,b einen schematischen Längsschnitt und eine schematische Draufsicht auf einen ersten Leiter mit distalseitiger LED eines möglichen Lichtapplikators einer beispielhaften Ausführungsform des hierin offenbarten Lichtapplikatorsystems;

Fig. 10a,b einen schematischen Längsschnitt und eine schematische Draufsicht auf einen ersten Leiter mit distalseitiger LED eines möglichen Lichtapplikators einer beispielhaften anderen Ausführungsform des hierin offenbarten Lichtapplikatorsystems;

Fig. 11a-d schematische Darstellungen eines Einführabschnitts vier weiterer beispielhafter Ausführungsformen eines hierin offenbarten Lichtapplikators;

Fig. 12a-c schematische Darstellungen der distalen Spitze eines möglichen Lichtapplikators einer beispielhaften Ausführungsform des hierin offenbarten Lichtapplikatorsystems;

Fig. 13 eine schematische Darstellung der distalen Spitze eines möglichen Lichtapplikators einer beispielhaften Ausführungsform des hierin offenbarten Lichtapplikatorsystems während des Betriebs;

Fig. 14 eine schematische Darstellung der distalen Spitze eines möglichen Lichtapplikators einer beispielhaften Ausführungsform des hierin offenbarten Lichtapplikatorsystems mit zwei verschiedenen Detailvarianten;

Fig. 15 eine schematische Darstellung der distalen Spitze eines möglichen Lichtapplikators einer anderen beispielhaften Ausführungsform des hierin offenbarten Lichtapplikatorsystems mit vier verschiedenen Detailvarianten;

Fig. 16 eine schematische Darstellung der distalen Spitze eines möglichen Lichtapplikators einer beispielhaften Ausführungsform des hierin offenbarten Lichtapplikatorsystems beim Einstich in die Haut eines Patienten;

Fig. 17 eine schematische Darstellung der distalen Spitze eines möglichen Lichtapplikators einer anderen beispielhaften Ausführungsform des hierin offenbarten Lichtapplikatorsystems beim Einstich in die Haut eines Patienten;

Fig. 18 eine schematische Darstellung der distalen Spitze eines möglichen Lichtapplikators einer anderen beispielhaften Ausführungsform des hierin offenbarten Lichtapplikatorsystems beim Einstich in die Haut eines Patienten;

Fig. 19 einen schematischen Querschnitt der distalen Spitze eines möglichen Lichtapplikators einer beispielhaften Ausführungsform des hierin offenbarten Lichtapplikatorsystems mit zwei verschiedenen Detailvarianten;

Fig. 20 eine schematische Darstellung eines Verstärkungselements einer distalen Spitze eines möglichen Lichtapplikators einer beispielhaften Ausführungsform des hierin offenbarten Lichtapplikatorsystems mit drei verschiedenen Detailvarianten;

Fig. 21 eine schematische Darstellung der distalen Spitze eines möglichen Lichtapplikators einer beispielhaften Ausführungsform des hierin offenbarten Lichtapplikatorsystems beim Einstich in die Haut eines Patienten;

Fig. 22 einen schematischen Querschnitt der distalen Spitze eines möglichen Lichtapplikators einer beispielhaften Ausführungsform des hierin offenbarten Lichtapplikatorsystems mit drei verschiedenen Detailvarianten;

Fig. 23 eine schematische Darstellung eines Verstärkungselements einer distalen Spitze eines möglichen Lichtapplikators einer beispielhaften Ausführungsform des hierin offenbarten Lichtapplikatorsystems;

Fig. 24 eine schematische Darstellung der distalen Spitze eines möglichen Lichtapplikators einer anderen beispielhaften Ausführungsform des hierin offenbarten Lichtapplikatorsystems mit Hautöffnung beim Einstich in fünf verschiedenen Phasen;

Fig. 25 eine schematische Darstellung der distalen Spitze eines möglichen Lichtapplikators einer anderen beispielhaften Ausführungsform des hierin offenbarten Lichtapplikatorsystems mit zwei verschiedenen Detailvarianten;

Fig. 26 eine schematische Darstellung der distalen Spitze eines möglichen Lichtapplikators einer anderen beispielhaften Ausführungsform des hierin offenbarten Lichtapplikatorsystems mit zwei verschiedenen Detailvarianten; und

Fig. 27 eine schematische Darstellung der distalen Spitze eines möglichen Lichtapplikators einer anderen beispielhaften Ausführungsform des hierin offenbarten Lichtapplikatorsystems.

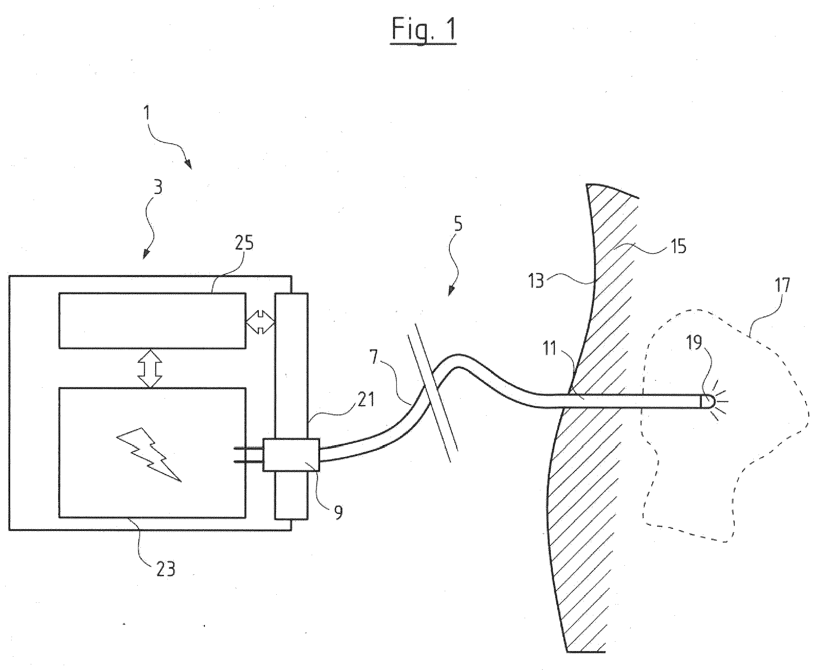

Fig. 1 zeigt ein Lichtapplikatorsystem 1 mit einer Lichtapplikatorbetriebseinheit 3 und einem auswechselbaren Lichtapplikator 5. Der Lichtapplikator 5 besteht im Wesentlichen aus einem flexiblen Kabelabschnitt 7, der sich von einem proximalseitigen Stecker 9 zu einem distalseitigen Einführabschnitt 11 erstreckt. Der Einführabschnitt 11 ist im Gegensatz zum Kabelabschnitt 7 der Abschnitt des Lichtapplikators 5, der dazu vorgesehen ist, in organisches Gewebe eines Körpers 13 eingestochen zu werden. Der Lichtapplikator 5 ist hier für die perkutane PDT geeignet und im PDT-Betrieb gezeigt. Der Einführabschnitt 11 ist hier als starrer Nadelabschnitt ausgestaltet, der beispielsweise CT- oder ultraschallunterstützt von außen durch die Haut 13 eines Patienten und darunterliegendes gesundes Gewebe 15 mit der distalen Spitze in pathologisches Körpergewebe 17, z. B. einen malignen Tumor, gestochen ist.

An der distalen Spitze weist der Einführ- bzw. Nadelabschnitt 11 eine distalseitige LED 19 zur In-Situ-Erzeugung von Anregungslicht für die PDT auf. Wird beispielsweise Chlorin e6 als Sensibilisator für die PDT eingesetzt, kann die dazu speziell ausgelegte LED 19 im Wellenlängenbereich von 660-670 nm Anregungslischt möglichst isotrop aussenden. Das Chlorin e6, das sich als Sensibilisator zuvor im pathologisches Körpergewebe 17 selektiv angereichert hat, produziert durch die Lichteinwirkung Sauerstoffradikale, die das pathologisches Körpergewebe 17 zerstören. Da sich das Chlorin e6 nicht im gesunden Gewebe 15 anreichert, bleibt das gesunde Gewebe 15 vom Licht im Wesentlichen unbeeinflusst.

Die LED 19 wird über einen elektrischen Leiter im Kabelabschnitt 7 und Einführabschnitt 11 von der Lichtapplikatorbetriebseinheit 3 mit Strom versorgt. Der proximalseitige Stecker 9 des Lichtapplikators 5 ist dazu in einen Anschluss 21 an der Lichtapplikatorbetriebseinheit 3 gesteckt. Zur Stromversorgung des Lichtapplikators 5 weist die Lichtapplikatorbetriebseinheit 3 ein elektrisches Versorgungsmodul 23 auf, das dazu eingerichtet ist, am Anschluss 21 einen bestimmten Betriebsstrom für den anschließbaren Lichtapplikator 5 bereitzustellen. Außerdem weist die Lichtapplikatorbetriebseinheit 3 ein Steuerungsmodul 25 auf, das dazu eingerichtet ist, den Stecker 9 zu identifizieren und das Versorgungsmodul 23 anzuweisen einen entsprechenden Betriebsstrom bereitzustellen,

Der Anschluss 21 kann beispielsweise eine Steckerleiste mit einer Mehrzahl von Steckplätzen sein, wobei als Identifikationsmittel in Form einer mechanischen Kennung nur bestimmte Steckerformen in bestimmte Steckplätze passen, sodass bei eingestecktem Stecker 9 eindeutig bestimmt ist, welcher Typ von Lichtapplikator 5 sich an einem Steckplatz befindet. Das Steuerungsmodul 25 kann dann festgelegte Betriebsparameter (Strom, Spannung, Betriebsdauer, usw.) pro Steckplatz vorsehen. Alternativ oder zusätzlich kann der Applikatortyp als Identifikationsmittel in Form einer signalbasierten Kennung vom Steuerungsmodul 25 auslesbar im Stecker 9 gespeichert sein und das Versorgungsmodul 23 entsprechend angesteuert werden. Bei einer signalbasierten Kennung kann sich der Lichtapplikator 5 selbst aktiv beim Steuerungsmodul 25 "anmelden" und identifizieren und/oder passiv vom Steuerungsmodul 25 abgefragt werden. Es kann auch eine vorhergehende Benutzung des Lichtapplikators 5 im Steuerungsmodul 25 und/oder im Stecker 9 abgespeichert sein, sodass nur eine einmalige Verwendung des Lichtapplikators 5 erlaubt werden kann.

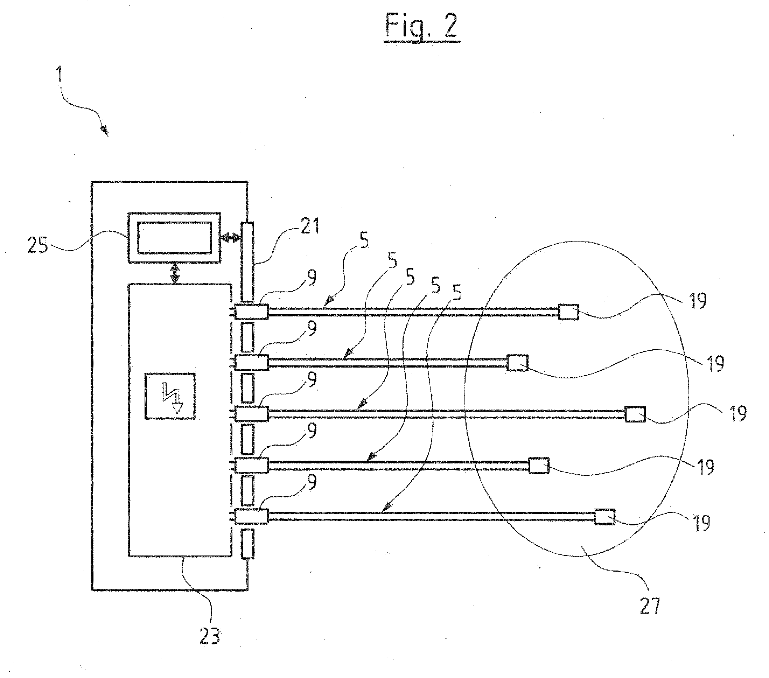

In Fig. 2 weist das Lichtapplikatorsystem 1 eine Mehrzahl von Lichtapplikatoren 5 auf, die zeitgleich an einer Mehrzahl an Anschlüssen 21 angeschlossen sind, um gemeinsam für eine PDT eines ganzen Organs 27 verwendet zu werden. Die Lichtapplikatoren 5 können alle gleichen Typs oder zumindest teilweise unterschiedlichen Typs sein. Die Lichtapplikatoren 5 sind an unterschiedlichen Stellen durch die Haut und mit unterschiedlichen Einstichtiefen verteilt in das Organ 27 eingestochen, sodass die LEDs 19 das Organ möglichst homogen mit Anregungslicht ausleuchten. Dies ist vor allem bei großen und/oder nicht genau lokalisierbaren Tumoren sinnvoll. Da gesundes Gewebe keinen Schaden durch die PDT erleidet, ist es sinnvoll, großzügig ein Volumen oder ein ganzes Organ um den Tumor auszuleuchten, um das Risiko zu verringern, dass pathologisches Gewebe untherapiert bleibt. Die einzelnen Lichtapplikatoren 5 können mit bestimmtem Typ, an bestimmter Position, mit bestimmtem Einstichwinkel und -tiefe beispielweise mittels einer auf die Haut geklebten oder anderweitig fixierten Hilfsschablone bzw. Template festgelegt sein. Die entsprechende Zuordnung der Lichtapplikatoren 5 zu den Anschlüssen 21 kann über die Identifikationsmittel festgelegt sein, sodass für eine festgelegte Art der PDT an einem bestimmten Organ 27 automatisch an jedem Anschluss 21 von dem Steuerungsmodul 25 die richtigen Betriebsparameter bereitgestellt werden. Das Fehlerrisiko bei einer komplexen Einstellung einer Mehrzahl an Lichtapplikatoren 5 kann somit verringert werden.

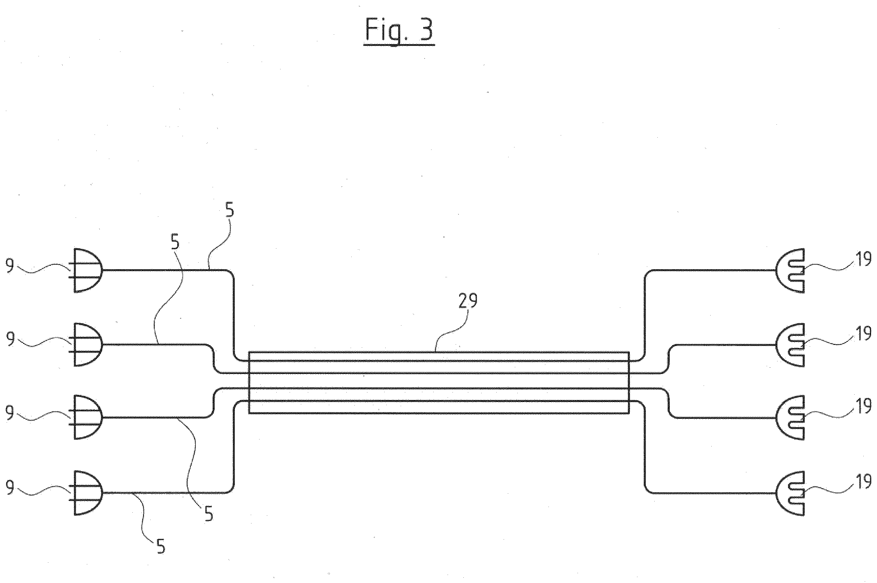

In Fig. 3 ist schematisch gezeigt, dass eine Mehrzahl von Lichtapplikatoren 5 für die interstitielle oder perkutane PDT im flexiblen Kabelabschnitt 7 zumindest abschnittsweise gebündelt sein kann, beispielsweise in Form eines mehradrigen Flachbandkabels 29. Die Lichtapplikatoren 5 können allerdings auch gebündelt oder einzeln durch einen oder mehrere Arbeitskanäle eines Endoskops oder Trokars geführt sein.

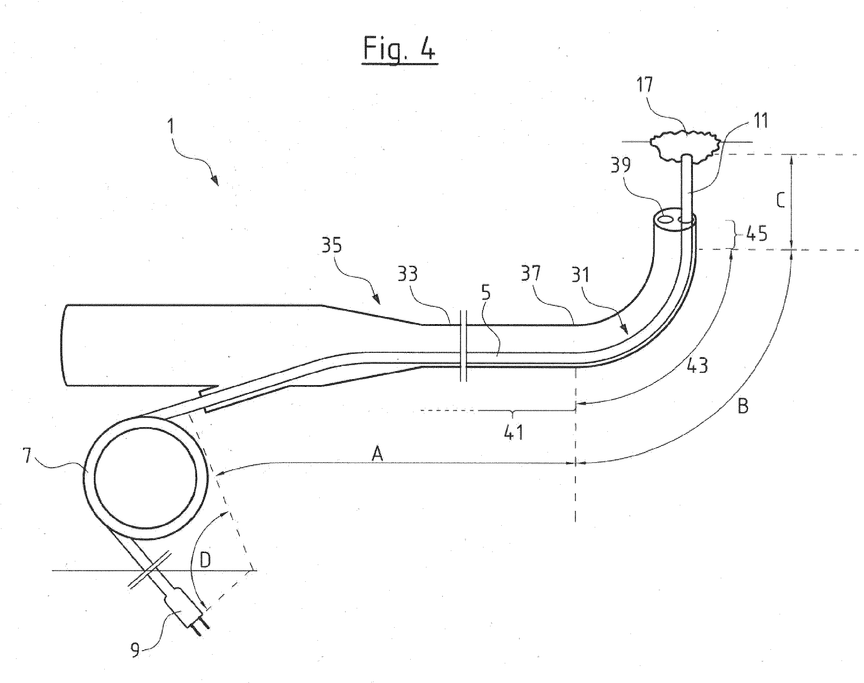

In Fig. 4 ist eine Ausführungsform eines Lichtapplikatorsystems 1 gezeigt, bei dem ein Lichtapplikator 5 für die interstitielle PDT durch einen Arbeitskanal 31 im Schaft 33 eines Schaftinstruments 35 in Form eines Endoskops geführt ist. Der Schaft 33 weist einen Einführabschnitt 37 auf, der dazu bestimmt ist, in den Körper eines Patienten eingeführt zu werden. Optional kann am distalen Ende des Einführabschnitt 37 ein Bildsensor 39 angeordnet sein, um ein Bild vom Inneren des Körpers des Patienten aufnehmen zu können. Der Einführabschnitt 37 weist einen ersten Schaftabschnitt 41, einen zweiten Schaftabschnitt 43 und einen dritten Schaftabschnitt 45 auf, wobei der erste Schaftabschnitt 41 proximal vom zweiten Schaftabschnitt 43 und der dritte Schaftabschnitt 45 distal vom zweiten Schaftabschnitt 43 verläuft. Der Schaft 33 ist im zweiten Schaftabschnitt 43 abwinkelbar und/oder weniger biegesteif als der erste und/oder dritte Schaftabschnitt 41, 45. Damit kann das distale Ende des Einführabschnitts 37 an verwinkelte Bereiche des Köperinneren, wie etwa Nierenkelche oder stark verzweigte Atemwege, minimalinvasiv platziert werden.

Entsprechend der Schaftabschnitte 41, 43, 45 weist auch der Lichtapplikator 5 verschiedene Lichtapplikatorabschnitte auf, nämlich einen ersten Lichtapplikatorabschnitt A, einen zweiten Lichtapplikatorabschnitt B und einem dritten Lichtapplikatorabschnitt C, wobei der erste Lichtapplikatorabschnitt A proximal vom zweiten Lichtapplikatorabschnitt B und der dritte Lichtapplikatorabschnitt C distal vom zweiten Lichtapplikatorabschnitt B verläuft. Der zweite Lichtapplikatorabschnitt B ist weniger biegesteif als der erste A und/oder dritte Lichtapplikatorabschnitt C. Der zweite Lichtapplikatorabschnitt B ist zumindest teilweise im zweiten Schaftabschnitt 43 angeordnet, wenn die distalseitige LED 19 des Lichtapplikators 5 am distalseitigen Ende des Einführabschnitts positioniert ist. Die Lichtapplikatorabschnitte A, B, C sind also in der Länge abgestimmt auf den Schaft 33 des Endoskops 35, damit einerseits der Lichtapplikator 5 dort biege- und torsionssteif genug ausgestaltet ist, um das distale Ende des Lichtapplikators 5 steuern zu können und andererseits dort flexibel genug ausgestaltet ist, um die Abwinkelbarkeit des Schafts im zweiten Schaftabschnitt 43 möglichst wenig zu beeinträchtigen. Der Lichtapplikator 5 hat in diesem Ausführungsbeispiel noch einen optionalen vierten Lichtapplikatorabschnitt D im proximalen flexiblen Kabelabschnitt 7, der proximalseitig vom ersten Lichtapplikatorabschnitt A und in gewissem Radius aufwickelbar außerhalb vom Endoskop 35 verläuft. Der distale Einführabschnitt 11 des Lichtapplikators 5 kann am distalen Ende des Arbeitskanals 31 herausragen und für die interstitielle PDT in pathologisches Gewebe 17 eingestochen werden. Der distale Einführabschnitt 11 des Lichtapplikators 5 kann dazu flexibel oder als Nadelabschnitt relativ biegesteif sein. Ein biegesteifer Nadelabschnitt 11 kann die Eindringtiefe in das Gewebe erhöhen, wogegen ein flexibler Einführabschnitt 11 besser durch einen abgewinkelten Arbeitskanal 31 schieb- oder ziehbar ist.

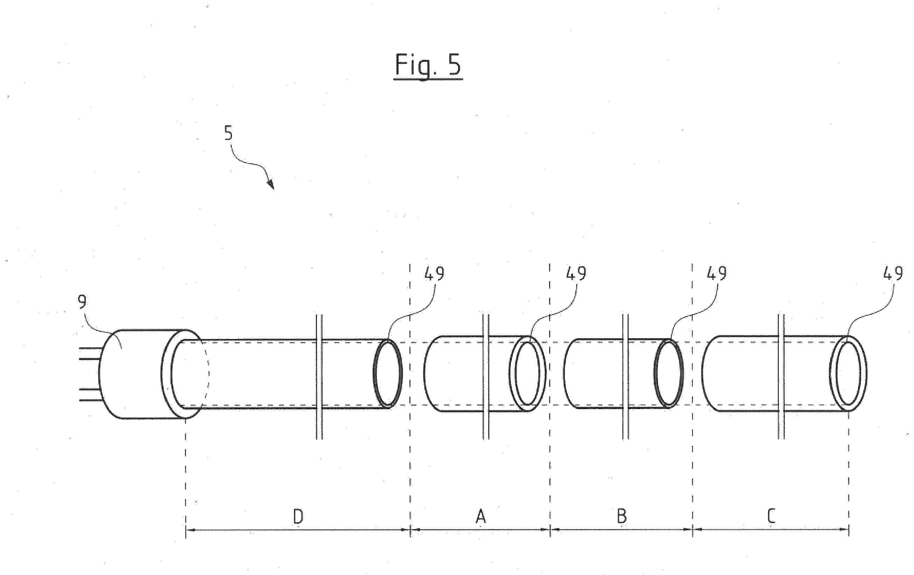

Wie in Fig. 5 gezeigt, ist der Lichtapplikator 5 im zweiten Lichtapplikatorabschnitt B dünner als in den Lichtapplikatorabschnitten A und C ausgestaltet, da eine geringere Biege- und Torsionssteifigkeit für kleine Abwinkelradien gebraucht wird. In den Lichtapplikatorabschnitten A und C ist er dicker, wo eine höhere Biege- und Torsionssteifigkeit vorteilhaft für die Handhabung ist. Es kann dabei die radiale Ausdehnung des elektrischen Leiters selbst und/oder seine isolierende Ummantelung den Erfordernissen für die lokale Biege- und Torsionssteifigkeit angepasst sein. Der Lichtapplikator 5 muss dabei nicht als Ganzes unterschiedlich dick in den verschiedenen Lichtapplikatorabschnitten A-D sein, sondern es kann beispielsweise nur ein versteifender Mantel unterschiedlich dick in den verschiedenen Lichtapplikatorabschnitten A-D sein. Eine sehr einfache Lösung ist es, eine radiale Ummantelung 49 als elektrischer Isolator und/oder Wärmedämmung abschnittsweise als Schläuche mit unterschiedlicher Wandstärke und/oder in unterschiedlicher Anzahl als zumindest teilweise miteinander überlappende Schläuche auszubilden und somit unterschiedliche Biege- und Torsionssteifigkeiten in den Lichtapplikatorabschnitten A-D zu erzielen. Die Schläuche können dabei als Schrumpfschläuche ausgeführt sein. Die Wandstärke der Ummantelung kann vergleichsweise dünn ausgestaltet werden, da die durch die Ummantelung bewirkte Erhöhung der Biegesteifigkeit mit dem Flächenträgheitsmoment $I_r$ korreliert. Beispielsweise bei einem Kreisring als Querschnittsform der Ummantelung gilt $I_r = \frac{\pi}{4}(R^4 - r^4)$ , sodass die Biegesteifigkeit mit der vierten Potenz des Radius skaliert, wobei R der Außenradius der Ummantelung und r der Innenradius der Ummantelung ist. Für einen Ummantelungsquerschnitt mit quadratischem Kastenprofil mit Außenbreite A und Innenbreite a gilt entsprechend $I_r = \frac{1}{12}(A^4 - a^4)$ . Selbst geringste Unterschiede der Dicke der Ummantelung zwischen den Lichtapplikatorabschnitten A bis D im Bereich von $10\,\mu m$ können daher einen signifikanten Einfluss auf die Biege- und Torsionssteifigkeit bewirken.

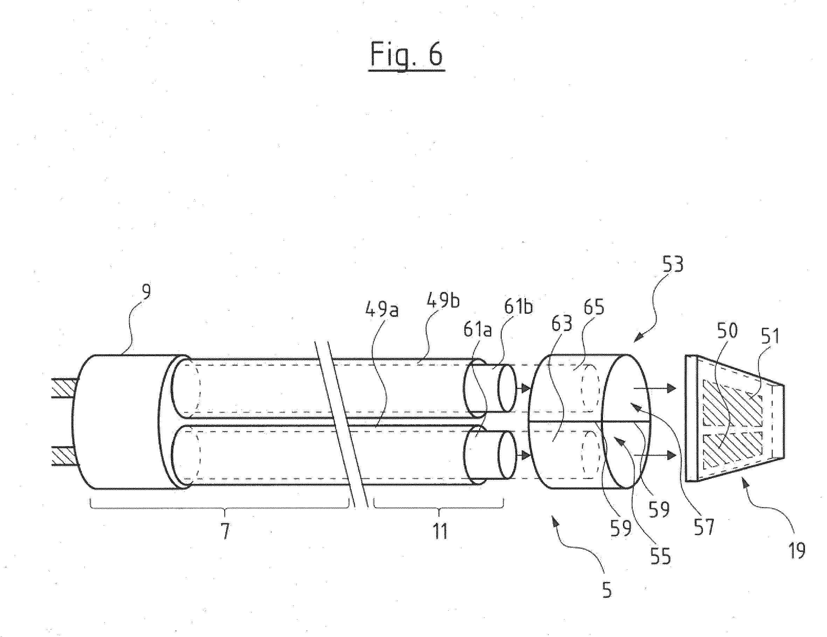

Fig. 6 zeigt eine Ausführungsform des Lichtapplikators 5, bei dem ein sogenannter Flip-Chip als LED 19 zum Einsatz kommt, bei dem sowohl der erste elektrische Kontakt 50 (hier Anodenkontakt) als auch der zweite elektrische Kontakt 51 (hier Kathodenkontakt) proximalseitig angeordnet sind. Der LED-Chip 19 ist hier beispielsweise mit einer dünnen Leitkleberschicht (nicht abgebildet) auf einem Adapterstück 53 aufgeklebt, das hinsichtlich seiner lateralen Abmessungen so ausgeführt ist, dass eine großflächige Anlage des LED-Chips 19 ermöglicht wird und damit ein guter proximalwärtiger Wärmefluss von der LED 19 zum Adapterstück 53 erzielt wird. Das Adapterstück 53 kann dabei wie in Fig. 6 aus zwei Metallhälften 55, 57 bestehen, die jeweils mit den Kontakten 50, 51 der LED in elektrisch leitendem Kontakt stehen und gegeneinander mittels einer dünnen Isolator-Schicht 59 elektrisch isoliert sind. Alternativ kann das Adapterstück 53 beispielsweise als einstückiges Keramikstück mit metallischen Durchführungen ausgeführt sein. Die LED wird über die Länge des Lichtapplikators 5 mit einem elektrischen Leiter 61 mit Strom versorgt, wobei hier der elektrische Leiter 61 als Zwillingslitze 61a,b ausgeführt ist, wobei jeweils eine der Zwillingslitzen 61a,b der Stromzuführung und die anderer der Zwillingslitzen 61b,a der Stromabführung dient. Das Adapterstück 53 weist proximalseitig Kontaktaufnahmen 63, 65 auf, mit welchen das distale Ende des elektrischen Leiters 61 elektrisch leitend verbindbar ist. Der elektrische Leiter 61 kann zumindest abschnittsweise flexibel oder starr ausgestaltet sein. Ob flexibel oder starr, es ist für den proximalwärtigen Wärmeabfluss in jedem Fall von Vorteil, wenn der elektrische Leiter 61 einen möglichst großen Querschnittsanteil, z.B. mindestens 70%, am Lichtapplikator 5 hat, da der elektrische Leiter 61 ein besserer Wärmeleiter ist als eine den elektrischen Leiter 61 umgebende isolierende Ummantelung 49a,b.

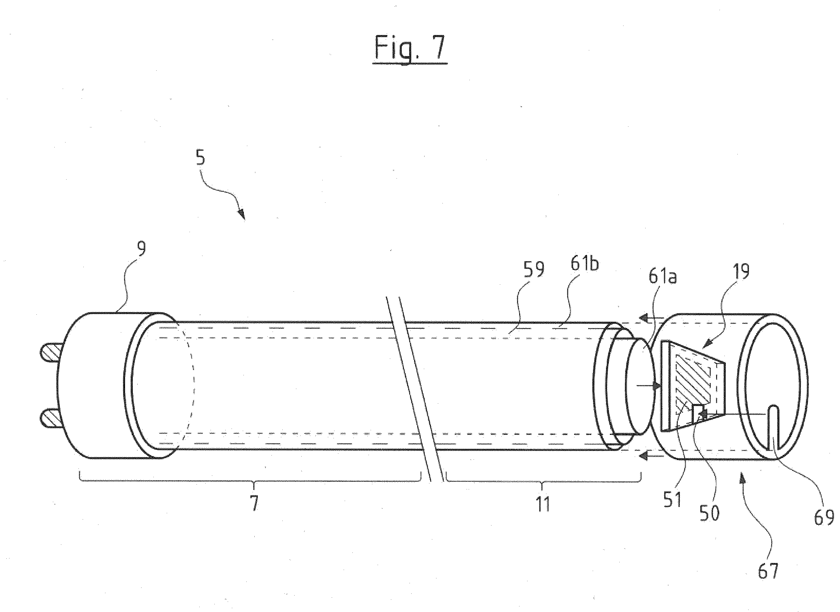

Im Gegensatz zum Ausführungsbeispiel gemäß Fig. 6 ist in Fig. 7 ein Lichtapplikator 5 gezeigt mit einem LED-Chip 19, der einen proximalseitig großflächigen zweiten elektrischen Kontakt 51, der gleichzeitig als Lichtreflektor in distale Richtung fungiert, und einen distalseitigen relativ kleinflächigen ersten elektrischen Kontakt 50 aufweist. Der elektrische Leiter 61 ist hier als Koaxialkabel ausgeführt, wobei dessen Seele 61a distalseitig mittels Leitkleber oder Lötzinn mit dem proximalseitigen zweiten elektrischen Kontakt 51 der LED 19 leitend verbunden ist. Für einen möglichst guten proximalwärtigen Wärmeabfluss von der LED 19 über die Seele 61a hat diese einen relativ großen Querschnittsflächenanteil am elektrische Leiter 61 bzw. am gesamten Lichtapplikator 5, z.B. mindestens 70%. Ein Außenleiter 61b und eine zwischen Außenleiter 61b und Seele 61a angeordnete Isolator-Schicht 59 sind entsprechend jeweils möglichst dünn ausgeführt. Nach außen hin ist der Außenleiter 61b von ebenfalls mindestens einer möglichst dünnen isolierenden Ummantelung (nicht in Fig. 7 gezeigt) umgeben. Wie in Fig. 5 kann über die Dicke und/oder Schichtenzahl der Ummantelung abschnittsweise die Biegesteifigkeit des Lichtapplikators 5 definiert sein. Die Seele 61a kann als relativ starrer massiver Draht oder als flexibles Litzenbündel ausgestaltet sein.

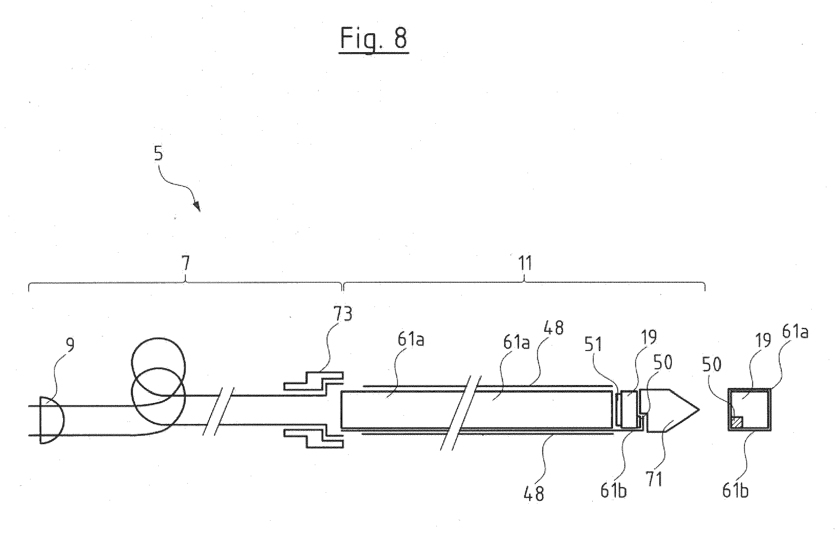

Der Außenleiter 61b in Fig. 7 dient als Rückleiter, der über eine distalseitige metallische Hülse 67, die über die LED 19 gestülpt ist, mit dem ersten elektrischen Kontakt 50 der LED 19 elektrisch leitend verbunden ist. Für den elektrischen Kontakt zwischen dem ersten elektrischen Kontakt 50 und der Hülse 67 ragt ein Kontaktbügel 69 der Hülse 67 radial nach innen. Wenngleich nicht in allen Figuren gezeigt, ist bei allen Ausführungsformen vorteilhafterweise distal von der LED eine lichtstrahlformende Komponente angeordnet, um eine gewünschte Abstrahlcharakteristik zu erzielen. Beispielsweise kann für die PDT eine isotrope Abstrahlung gewünscht sein, sodass ein Streukörper 71 (siehe Figuren 8-10 und 13-27) die lichtstrahlformende Komponente bilden kann. Falls eine Fokussierung des Lichts gewünscht ist, kann eine Linse als lichtstrahlformende Komponente distalseitig vom LED-Chip 19 angeordnet sein.

In Fig. 8 ist eine Ausführungsform des Lichtapplikators 5 gezeigt, die besonders vorteilhaft für die perkutane PDT ist. In Fig. 8 ist rechts separat eine Draufsicht auf die LED 19 gezeigt. Der proximale Kabelabschnitt 7 des Lichtapplikators 5 mit proximalseitigem Stecker 9 zum Anschluss an eine Lichtapplikatorbetriebseinheit 3 ist flexibel und weist distalseitig einen lösbaren Verbindungsanschluss 73 für einen relativ starren distalen Nadelabschnitt 11 auf. Der Nadelabschnitt 11 dient ganz oder teilweise als Einführabschnitt zum Einstechen in Gewebe eines Körpers. Der Nadelabschnitt 11 erhält seine Biegesteifigkeit hauptsächlich durch einen zentralen, massiven, starren, stabförmigen ersten elektrischen Leiter 61a. Analog zu Fig. 7 ist distalseitig am ersten elektrischen Leiter 61a eine LED 19 verbunden. Aus der Draufsicht auf die LED 19 rechts in Fig. 8 wird deutlich, dass der elektrische Leiter 61a wie die LED 19 einen im Wesentlichen quadratischen Querschnitt hat, der nur wenig größer ist als die LED 19. Der Querschnitt der momentan am Markt erhältlichen oder produzierbaren kleinstmöglichen LED 19 setzt hier eine untere Grenze für die Miniaturisierung des Lichtapplikators 5, der möglichst minimalinvasiv, also dünn, ausgestaltet ist und somit möglichst wenig über die lateralen Abmessungen der LED 19 hinausragen sollte. So können momentan Lichtapplikatorquerschnitte von deutlich weniger als 1 mm² erzielt werden, beispielsweise 0,25 mm² und weniger.

Ein zweiter elektrischer Leiter 61b kann als Rückleiter in Form einer gegen den ersten elektrischen Leiter 61a isolierten sehr dünnen Flexplatine oder eines Lackdrahts an einer Seite des ersten elektrische Leiters 61a entlanggeführt und an seinem distalen Ende L-förmig gefaltet oder gebogen sein, um den distalseitigen Anodenkontakt 50 der LED 19 zu kontaktieren. Die metallische Hülse 67 aus Fig. 7 kann dann in beiden Fällen entfallen. Die Leiter 61a, 61b sind hier mit einer dünnen Ummantelung 49 ummantelt, die vorzugsweise aus Kunststoff als Schrumpfschlauch biokompatibel ausgebildet ist und das Gleitverhalten des Nadelabschnitts 11 durch Köpergewebe verbessert. Außerdem dient die Ummantelung 49 als elektrische Isolierung und Wärmedämmung nach außen.

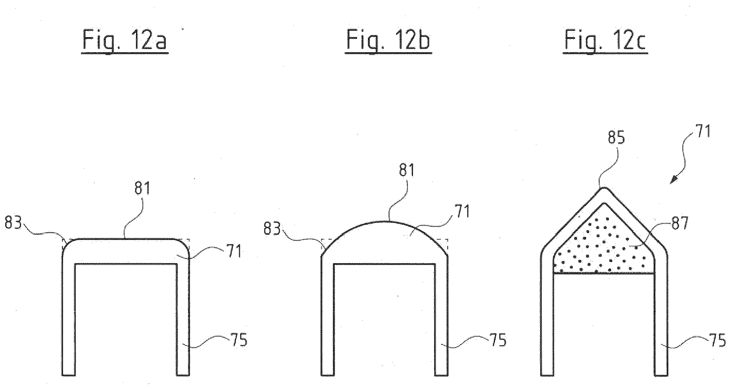

In Fig. 8 ist eine distal von der LED 19 angeordnete lichtstrahlformende Komponente in Form eines lichttransparenten Streukörpers 71 gezeigt, der als eine sich distalwärts verjüngende Nadelspitze des Nadelabschnitts 11 fungiert. In Figuren 12 bis 27 sind vorteilhafte Ausgestaltungen der Nadelspitze näher gezeigt.

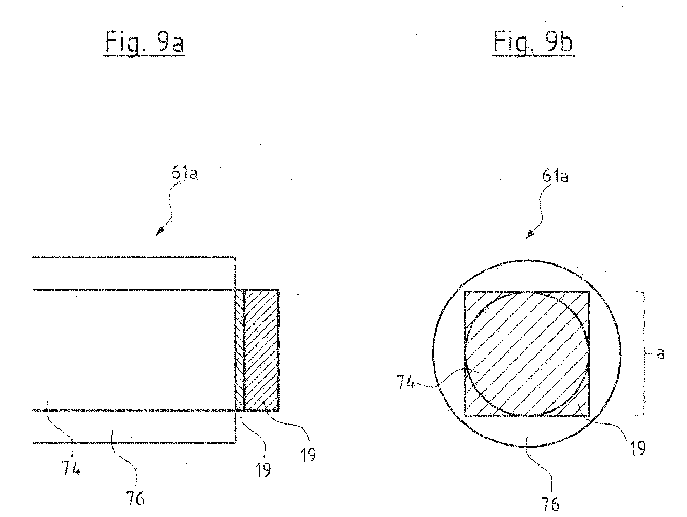

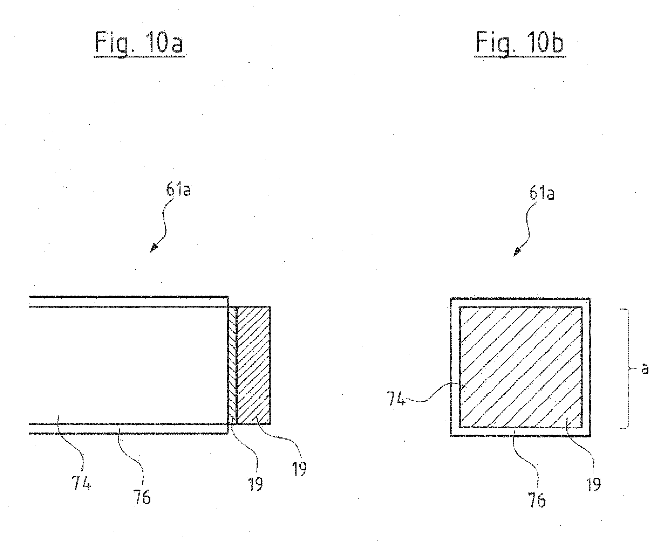

Um bei Querschnitten von unter 1 mm² eine hohe Biegesteifigkeit des ersten elektrischen Leiters 61a zu erzielen, ist es vorteilhaft, ein Material mit möglichst hohem Elastizitätsmodul als erster elektrischer Leiter zu verwenden. Beispielsweise Stahl hat mit über 200 MPa einen hohen Elastizitätsmodul. Allerdings hat Stahl den Nachteil, dass seine Wärmeleitfähigkeit deutlich unter 100 W/(Km) bei 20°C liegt, üblicherweise im Bereich von 50 W/(Km) bei 20°C. Da der erste elektrische Leiter 61a nicht nur biegesteif, sondern auch möglichst gut die Abwärme der LED proximalwärts ableiten soll, wäre zur Wärmeleitung ein erster elektrischer Leiter 61a aus Kupfer oder Silber mit einer Wärmeleitfähigkeit von mehr als 400 W/(Km) bei 20°C sinnvoll, oder zumindest aus Aluminium mit einer Wärmeleitfähigkeit von mehr als 200 W/(Km) bei 20°C. Allerdings haben Kupfer, Aluminium und Silber mit unter 100 MPa einen wesentlich geringeren Elastizitätsmodul als Stahl. Zur Erzielung einer hohen Biegesteifigkeit bei gleichzeitig hoher Wärmeleitfähigkeit ist der elektrische Leiter 61a, wie in Figuren 9a,b und Fig. 10a,b gezeigt, mit einem Kupferkern 74 und einem Stahlmantel 76 ausgeführt. Der Stahlmantel 76 kann relativ dünnwandig im Vergleich zum Radius des Kupferkerns 74 sein, da der Versteifungseffekt wie oben beschrieben in vierter Potenz mit dem Radius skaliert. Gleichzeitig ist die proximalwärtige Wärmeleitung besser je größer der Querschnitt des Kupferkerns 74 ist. Für eine möglichst gute Wärmeableitung ist der Durchmesser des Kupferkerns 74 an die lateralen Abmessungen der LED 19 angepasst. In Figuren 9a,b ist der Querschnitt des Leiters 61 rund und die LED 19 in etwa quadratisch mit einer Seitenlänge a, sodass der Durchmesser des Kupferkerns 74 in etwa a beträgt. Der Stahlmantel 76 ist hier mindestens ($\sqrt{2} - 1$) a dick, damit die Ecken der LED 19 nicht lateral über den Leiter 61 hinausragen. In Figuren 10a,b ist der Querschnitt des Leiters 61 wie die LED 19 in etwa quadratisch, wobei der Querschnitt des Kupferkerns 74 in etwa dem Querschnitt der LED 19 entspricht. Der Stahlmantel 76 ist hier wesentlich dünner als in Figuren 9a,b, z.B. weniger als 0,15 ▪ a dick.

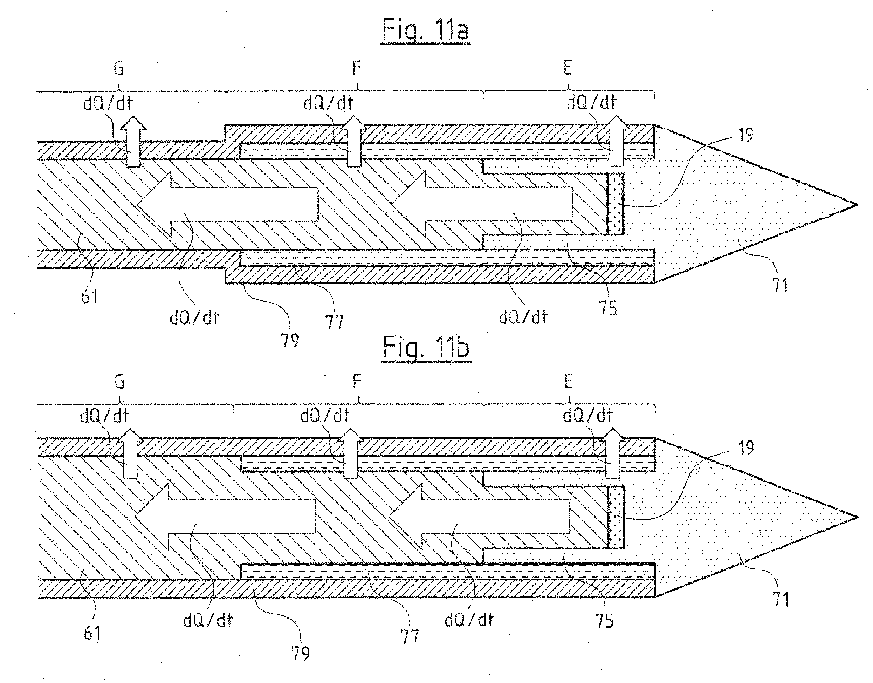

in Figuren 11a und 11b wird das Wärmemanagement im Einführabschnitt 11 des Lichtapplikators 5 deutlich. Der Einführabschnitt 11 kann hier starr oder flexibel sein, also der elektrische Leiter 61 als massiver Stab oder als flexibles Litzenbündel ausgebildet sein. Es besteht eine technische Herausforderung darin, bei kleinen Querschnitten, insbesondere Querschnitten von unter 1 mm², die Abwärme der LED 19 einerseits so gut abzuleiten, dass die LED 19 keinen Schaden nimmt und auch mit voranschreitender Einschaltzeit der LED die Höhe der abgegebenen optischen Leistung aufrecht erhalten werden kann, da mit ansteigender LED-Temperatur die abgegebene optische Leistung absinkt. Anderseits darf das umgebende Körpergewebe nicht so stark erhitzt werden, dass es Schaden nimmt. Eine zu starke Wärmedämmung schadet der LED 19 und eine zu schwache Wärmedämmung schadet aufgrund eines extrem inhomogenen radialen Wärmeflusses und eines vor allen Dingen im Bereich der LED 19 sehr stark überhöhten radialen Wärmeflusses vom Applikator an das umgebende Gewebe dort dem Gewebe. Zur Lösung dieses Problems nimmt die radiale Wärmedämmung proximalwärts von der LED 19 ab. In den gezeigten Ausführungsbeispielen geschieht dies stufenweise zwischen mehreren Wärmedämmungsabschnitten E, F, G. In einem ersten distalen Wärmedämmungsabschnitt E, der die LED 19 radial umgibt, ist die radiale Wärmedämmung am stärksten , sodass in diesem Bereich des Einführabschnitts 11, also im Bereich der LED 19 als Wärmequelle, wo der Wärmefluss dQ/dt im Lichtapplikator 5 insgesamt sehr hoch ist, weil dort fast noch keine Wärme an die äußere Umgebung abgegeben werden konnte, durch eine gute radiale Wärmedämmung der radiale Wärmefluss dQ/dt vom Lichtapplikator 5 zum Gewebe auf ein moderates Maß gedämpft wird, um das umliegende Gewebe nicht zu schädigen. Dazu weist die Wärmedämmung im distalen Wärmedämmungsabschnitt E drei Schichten auf.

Radial ganz innen wirkt als erste Wärmedämmschicht 75 im ersten distalen Wärmedämmungsabschnitt E ein sich hülsenförmig um die LED 19 erstreckender proximaler Abschnitt 75 der Nadelspitze 71, die distal von der LED 19 einen lichttransparenten Streukörper bildet. Der Streukörper 71 bewirkt eine möglichst isotrope Abstrahlung des Lichts in einen möglichst großen Raumwinkel. Die erste Wärmedämmschicht 75 weist wie der Streukörper 71 selbst einen Kunststoff, beispielsweise Epoxidharz, als Hauptbestandteil auf. Kunststoffe sind vorteilhafterweise durch eine vergleichsweise geringe Wärmeleitfähigkeit gekennzeichnet. Als zweite Wärmedämmschicht umgibt im ersten distalen Wärmedämmungsabschnitt E ein erster Schrumpfschlauch 77 aus Kunststoff, beispielsweise Polyethylenterephthalat (PET), die erste Wärmedämmschicht 75. Als dritte Wärmedämmschicht umgibt im ersten distalen Wärmedämmungsabschnitt E ein zweiter Schrumpfschlauch 79 aus Kunststoff, beispielsweise Polyethylenterephthalat (PET), die zweite Wärmedämmschicht 77.

Vom ersten distalen Wärmedämmungsabschnitt E erstreckt sich proximalwärts ein zweiter Wärmedämmungsabschnitt F, der aus der zweiten Wärmedämmschicht 77 und der dritten Wärmedämmschicht 79 besteht. Die erste Wärmedämmschicht 75 aus dem proximalen Hülsenabschnitt der Nadelspitze 71 erstreckt sich nicht bis in den zweite Wärmedämmungsabschnitt F. Dadurch kann der Leiter 61 die Wärme besser radial abgeben als im ersten Wärmedämmungsabschnitt E, sodass ein Wärmestau vermieden wird und ein proximalwärtiger Wärmeabfluss von der LED 19 durch den Leiter 61 gewährleistet bleibt. Vom zweiten distalen Wärmedämmungsabschnitt F erstreckt sich proximalwärts ein dritter Wärmedämmungsabschnitt G, der nur aus der dritten Wärmedämmschicht 79 besteht. Die zweite Wärmedämmschicht 77 erstreckt sich nicht bis in den dritten Wärmedämmungsabschnitt G. Dadurch kann der Leiter 61 die Wärme noch besser radial abgeben als im zweiten Wärmedämmungsabschnitt F, sodass auch hier ein Wärmestau vermieden wird und ein proximalwärtiger Wärmeabfluss von der LED 19 durch den Leiter 61 gewährleistet bleibt. Im Ergebnis bewirkt die proximalwärts von der LED 19 abnehmende radiale Wärmedämmung eine Verteilung des radialen Wärmeflusses über die Länge des Einführabschnitt 11, sodass der longitudinale Temperaturgradient ΔT/ΔL am Außenradius der dritten Wärmedämmschicht 79 über die Wärmedämmungsabschnitte E, F, G möglichst gering ist und somit gewebeschädigende Temperaturspitzen vermieden werden. Wie durch die Größe der weißen Blockpfeile in Fig. 1 1a,b angedeutet, wird der Unterschied im radialen Temperaturgradient ΔT/Δr zwischen den Wärmedämmungsabschnitten E, F, G durch die unterschiedliche Dicke der radialen Wärmedämmung und die daraus resultierenden unterschiedlichen radialen thermischen Widerstände der Wärmedämmungsabschnitte E, F, G möglichst ausgeglichen, sodass die jeweils durch die Wärmedämmungsabschnitte E, F, G radial nach außen übertragene Wärmeleistung dQ/dt möglichst gleich ist.

In den Figuren 11a und 11b wird die Stärke der Wärmedämmung hauptsächlich über die Gesamtdicke der Wärmedämmung bestimmt. Wie in Fig. 11a gezeigt, kann der Lichtapplikator 5 daher proximalwärts dünner ausgestaltet werden. Dies ist allerdings kein großer Vorteil, da der maximale Querschnitt des Lichtapplikators 5 durch den ersten Wärmedämmschicht E mit drei Wärmedämmschichten bestimmt ist. In Fig. 11b ist gezeigt, dass sich entsprechend der proximalwärts abnehmenden Dicke der Wärmedämmung der Querschnitt des Leiters 61 vergrößern kann, sodass der Querschnitt des Lichtapplikator 5 über die Wärmedämmungsabschnitte E, F, G hinweg im Wesentlichen konstant ist. Damit wird die proximalwärtige Wärmeleitung dQ/dt durch den Leiter 61 entsprechend verbessert.

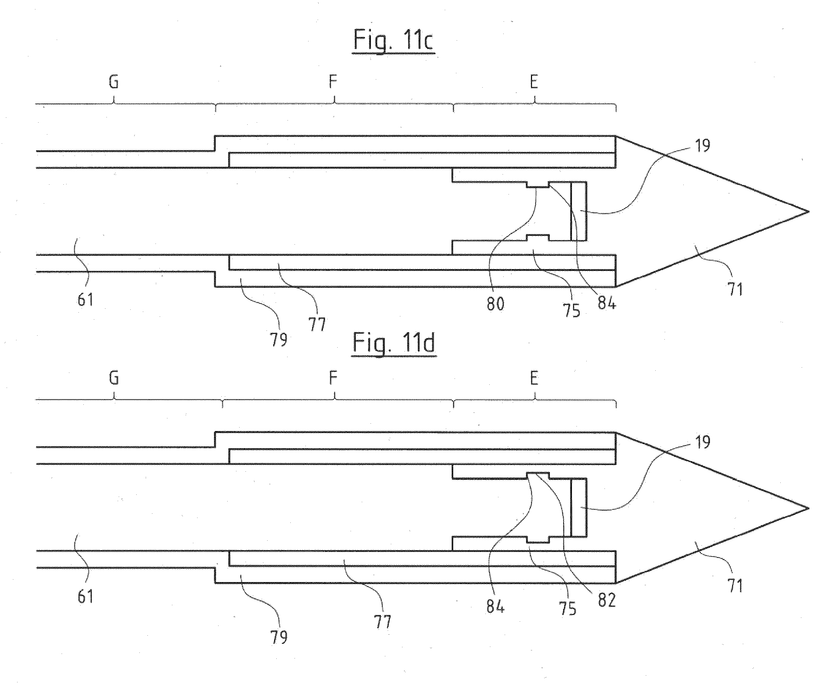

In den Figuren 11c und 11d ist gezeigt, wie eine zusätzliche Verbesserung der Befestigungssicherheit für die Nadelspitze 71 erreicht werden kann. Der Leiter 61 weist dazu in einem distalen Endabschnitt außenseitig mindestens eine Vertiefung 80 (siehe Fig. 11c) und/oder Aufweitung 82 (siehe Fig. 11d) auf, beispielweise als lokale Querschnittsverjüngung und/oder -vergrößerung. Dadurch wird erreicht, dass der hülsenförmige proximale Abschnitt 75 der Nadelspitze 71 mit dem ersten Leiter 61 einen Formschluss bildet. Die Nadelspitze 71 kann als Kunststoffgussteil oder als Kunststoffspritzgussteil ausgebildet sein, das beispielsweise durch Umspritzen oder Umgießen mit der außenseitigen Vertiefung 80 oder Aufweitung 82 des Leiters 61 einen Formschluss mittels eines Hinterschnitts 84 bilden kann.

In den Figuren 12 bis 27 sind verschiedene Ausgestaltungen der Nadelspitze bzw. des Streukörpers 71 gezeigt. Figuren 12a-c zeigen jeweils einen prinzipiellen hülsenförmigen Aufbau einer strahlformenden Komponente distalseits der LED (nicht gezeigt). In Fig. 12a ist die Nadelspitze im Wesentlichen topfförmig mit ebener Stirnfläche 81 und leicht abgerundeten Kanten 83. Die Nadelspitze in Fig. 12a ist daher relativ stumpf. Dies kann vorteilhaft sein, wenn kein Gewebe geschnitten, sondern nur weggedrückt werden soll. Für die perkutane PDT ist solch eine Nadelspitze allerdings wegen des hohen Widerstands beim Einstechen weniger geeignet. In Fig. 12b ist die Stirnfläche 81 komplett abgerundet, was einerseits den Widerstand beim Einstechen gegenüber der ebenen Stirnfläche 81 aus Fig. 12a verringert und andererseits einen Linseneffekt erzielt. In Fig. 12c verjüngt sich die Nadelspitze distalwärts kegelförmig zu einer relativ stumpfen Spitze 85. Das Kegelvolumen ist hier zum Großteil mit einem lichtstreuenden Material 87 ausgefüllt.

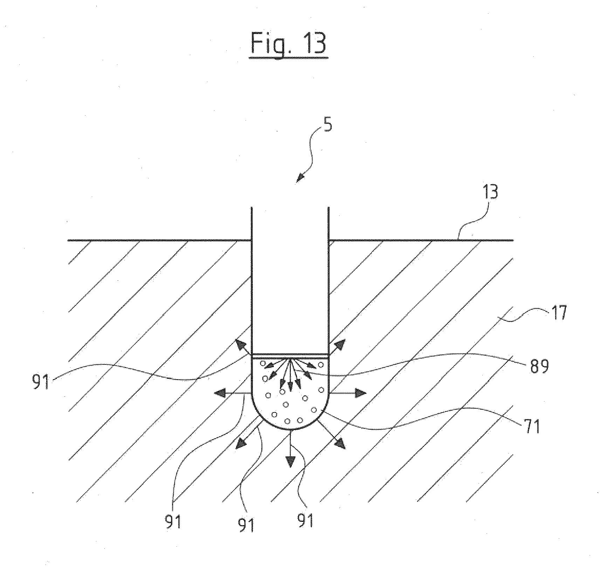

Fig. 13 zeigt einen perkutan (durch die Haut 13) in pathologisches Gewebe 17 eingestochenen Lichtapplikator 5 mit distalseitiger Nadelspitze, die als kuppelförmiger Streukörper 71 ausgebildet ist. Der Streukörper 71 streut das von der LED 19 gemäß der Abstrahlcharakteristik eines Lambert'schen Strahlers abgestrahlte Licht 89 derart, dass das den Streukörper 71 verlassende Licht 91 möglichst isotrop in einen möglichst großen Raumwinkel abgestrahlt wird. Das vom Streukörper 71 abgestrahlte Licht hat dadurch teilweise auch proximalwärts gerichtete Richtungskomponenten.

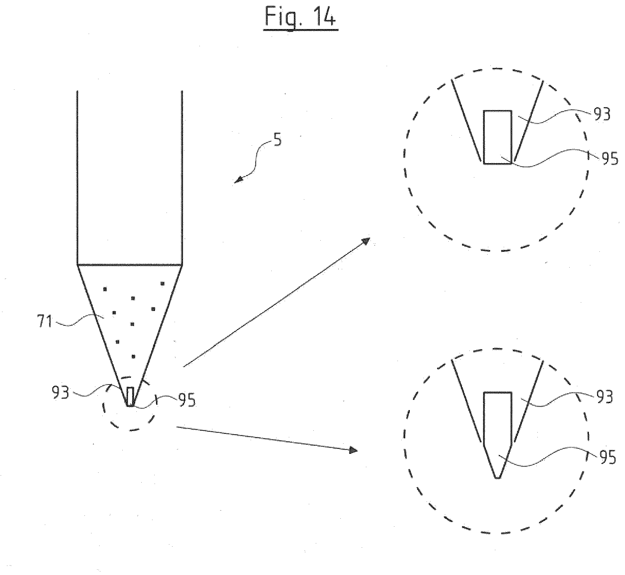

Bei der interstitiellen oder perkutanen PDT soll die mechanische Einwirkung auf Haut 13 und Gewebe 15, 17, insbesondere auf gesundes Gewebe 15, das ggf. auf dem Weg zum pathologischen Gewebe 17 passiert werden muss, möglichst minimalinvasiv sein. Es ist das vorrangige Ziel, dass der Einstich keine dauerhaften Schädigungen der Haut 13 und des gesunden Gewebes 15 herbeiführt. Außerdem soll der Einstich möglichst schnell verheilen und keine oder kleinstmögliche Narben hinterlassen. Zu diesem Zweck kann es vorteilhaft sein, die Spitze des Lichtapplikators 5 besonders spitz und/oder scharf auszugestalten. Dabei kann es allerdings technisch schwierig sein, die distale Spitze und/oder die Kante scharf genug aus dem lichttransparenten Material des Streukörpers 71 herstellen zu können. Beispielsweise ist Epoxidharz wie die meisten anderen Kunststoffe relativ weich und kann sich beim Einstich an der Spitze und/oder Kante biegen. Härteres lichttransparentes Material wie etwa Quarzglas ist zwar biegesteifer, jedoch ist die Bearbeitung deutlich aufwändiger und dadurch mit wesentlich höheren Kosten verbunden. Außerdem sind härtere transparente Werkstoffe wie beispielsweise Quarzglas spröde, sodass das Risiko von Absplitterungen sehr hoch wäre. Zur Lösung dieses Problems weist, wie in Fig. 14 gezeigt, ein Nadelspitzenendbereich 93 des beispielsweise kegel- oder pyramidenförmigen Streukörpers 71 ein Verstärkungselement 95 auf. Das Verstärkungselement 95 ist aus hartem Metall, wie etwa Stahl, und zumindest teilweise in ein relativ weiches Streukörpermaterial, wie etwa Epoxidharz eingebettet. Im Ausführungsbeispiel von Fig. 14 ist das Verstärkungselement 95 als relativ kurzer Dorn ausgeführt, der zur Verstärkung des Nadelspitzenendbereichs 93 zur Erhöhung sowohl der Biegesteifigkeit als auch der Biegefestigkeit ganz eingebettet sein kann (siehe Detailansicht rechts oben in Fig. 14) oder zur Schärfung der Nadelspitze distal aus dem Streukörper 71 herausragt (siehe Detailansicht rechts unten in Fig. 14). Die Querschnittsfläche des Verstärkungselements 95 ist relativ klein, damit es distalwärtig möglichst wenig Schatten wirft, Dazu ist es auch von Vorteil, das Verstärkungselements 95 möglichst kurz auszugestalten, wohingegen der Versteifungseffekt des Nadelspitzenendbereich 93 größer ist bei längerer Ausführung des Dorns 95.

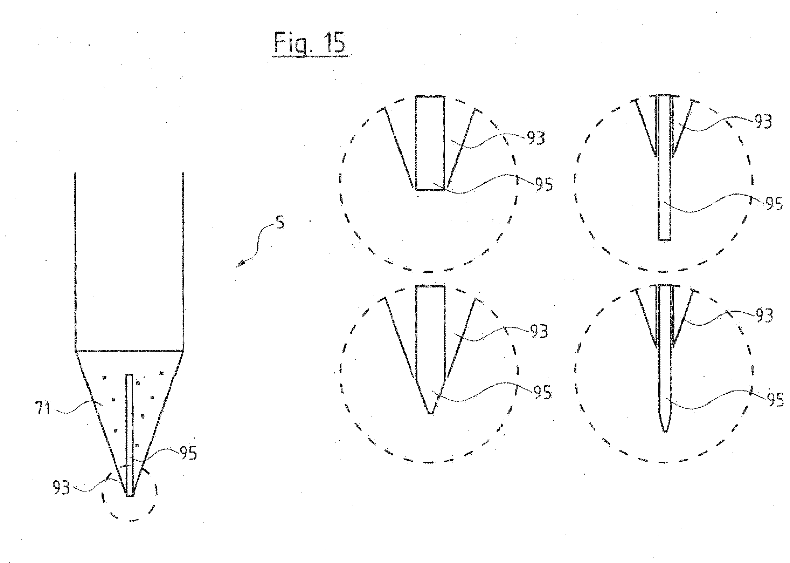

In Fig. 15 ist solch eine längere Ausführung des Dorns 95 als Verstärkungselement gezeigt, Bei dünner Ausführung ist der etwas größere Schattenwurf im Vergleich zum kurzen Dorn 95 aus Fig. 14 vernachlässigbar. Durch die tiefere Einbettung des Dorn 95 in den Streukörper 71 ist der Dorn 95 besser stabilisiert und kann ggf. weiter distalwärts aus dem Streukörper 71 herausragen (siehe Detailansichten rechts in Fig. 15).

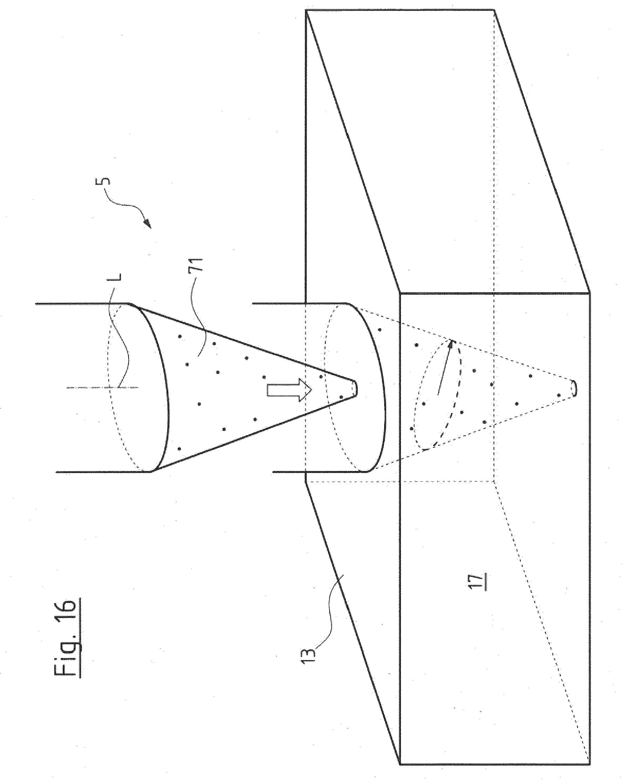

In Fig. 16 ist das prinzipielle perkutane (durch die Haut 13) Einstechen eines Lichtapplikators 5 in Gewebe 17 verdeutlicht. Mit der sich distalwärts verjüngenden Nadelspitze wird die durchstochene Haut 13 und das Gewebe 17 so weit aufgeweitet, dass der Lichtapplikators 5 hindurch passt. Je spitzer die Nadelspitze ist, desto behutsamer geschieht diese Aufweitung der Haut 13 und des Gewebes 17 und desto geringer ist die Kraft, die der Anwender dazu aufbringen muss.

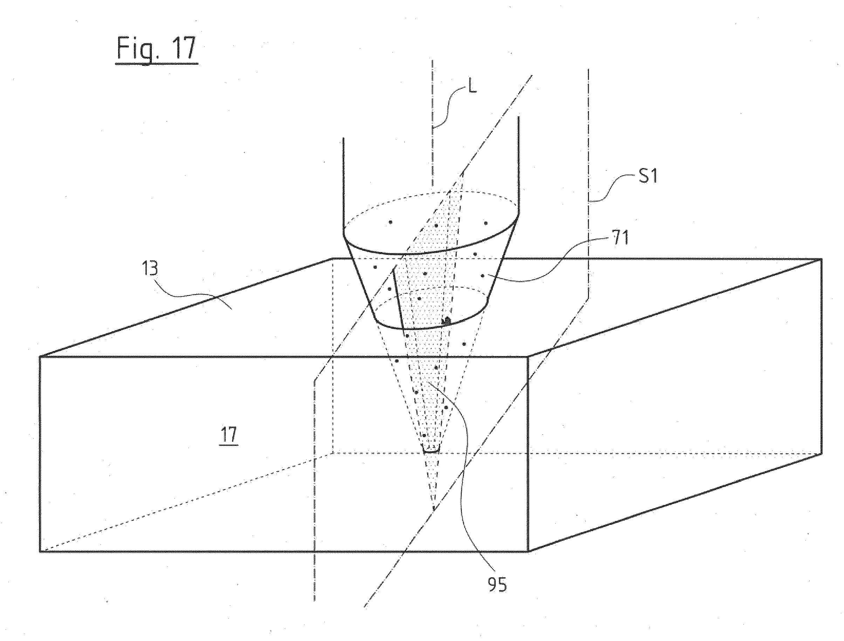

In Fig. 17 ist das Verstärkungselement 95 als dreieckige dünne scharfe Klinge ausgeführt, die in den kegelförmigen Streukörper 71 eingebettet ist und sowohl distal als auch lateral aus dem Streukörper 71 herausragt. Die Klinge 95 definiert eine Längsschnittebene S 1, die parallel zur Längsachse L des Lichtapplikators 5 verläuft. Die Klinge 95 durchsticht und zerschneidet beim Einstich des Lichtapplikators 5 das zu passierende Gewebe, um die Gewebeaufweitung zu erleichtern und den Widerstand zu reduzieren. Insbesondere bei festem Gewebe 17 und für das Durchdringen zäher Hautschichten ist dies sinnvoll.

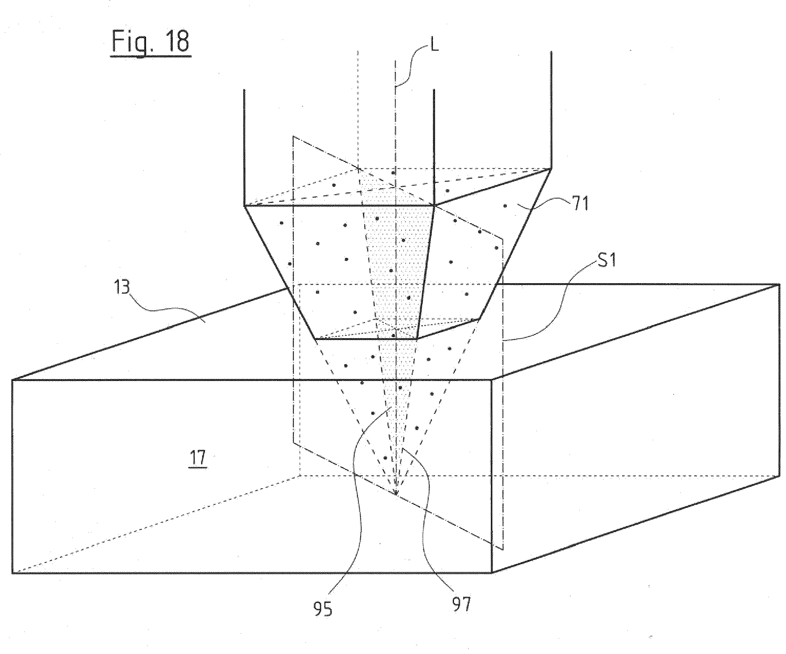

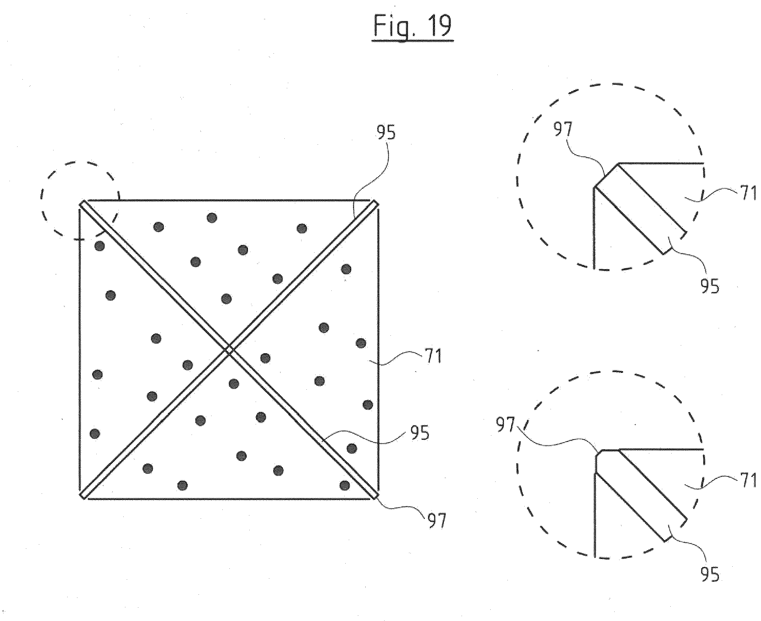

In Fig. 18 ist eine Ausführungsform gezeigt, bei der der Streukörper 71 pyramidenförmig mit im Wesentlichen quadratischer Grundfläche ist. Wie in Figur 19 hat der Einführabschnitt 11 des Lichtapplikators 5 hier einen quadratischen Querschnitt. Das Verstärkungselement 95 ist hier ebenfalls als dreieckige dünne scharfe Klinge ausgeführt, die jedoch vollständig in den kegelförmigen Streukörper 71 eingebettet ist und nicht oder kaum distal oder lateral aus dem Streukörper 71 herausragt. Die Längsschnittebene S1, in der die Klinge 95 liegt, wird hiervon der Diagonalen der quadratischen Grundfläche des Streukörpers 71 und der Längsachse L des Lichtapplikators 5 aufgespannt. Die Kanten 97 des Streukörpers 71 bilden hier von der Klinge 95 verstärkte Schnittkanten (siehe Detailansichten rechts in Fig. 19). In Fig. 19 ist das Verstärkungselement als zwei im Querschnitt im rechten Winkel gekreuzt zueinander angeordnete Klingen gezeigt. Es entsteht dadurch beim Einstechen ein Kreuzschnitt, mit dem sich das Gewebe besonders leicht aufweiten lässt.

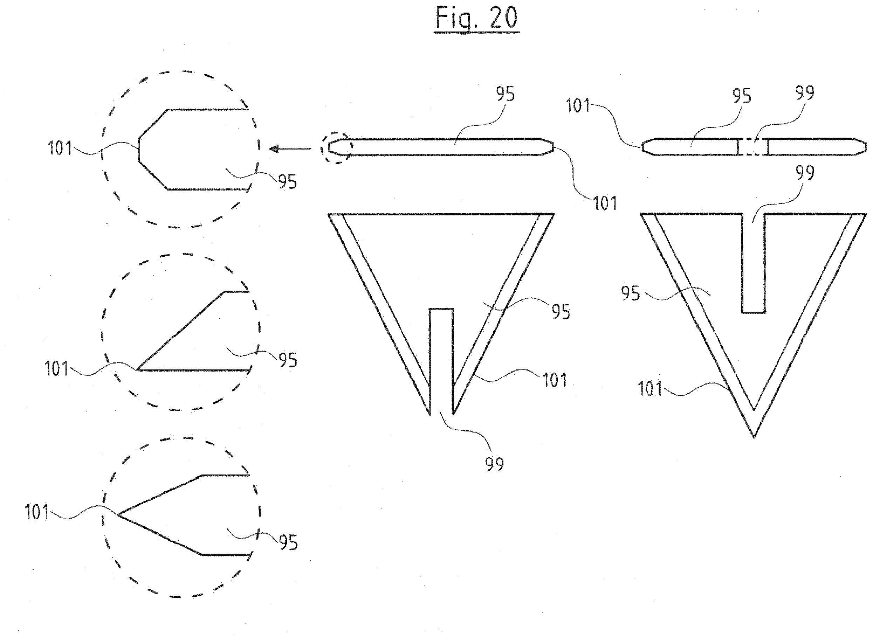

Wie in Fig. 20 gezeigt, können die gekreuzten Klingen 95 auf verschiedene Weise am Schneidrand scharf geschliffen sein. Die beiden Klingen 95 können jeweils korrespondierende Schlitze 99 aufweisen, sodass die Klingen gekreuzt ineinandergesteckt werden können. Die Schlitze 99 erstrecken sich jeweils in Längsrichtung in etwa über die Hälfte der Klingen 95. Die Breite der Schlitze 99 entspricht in etwa der Dicke der Klingen 95.

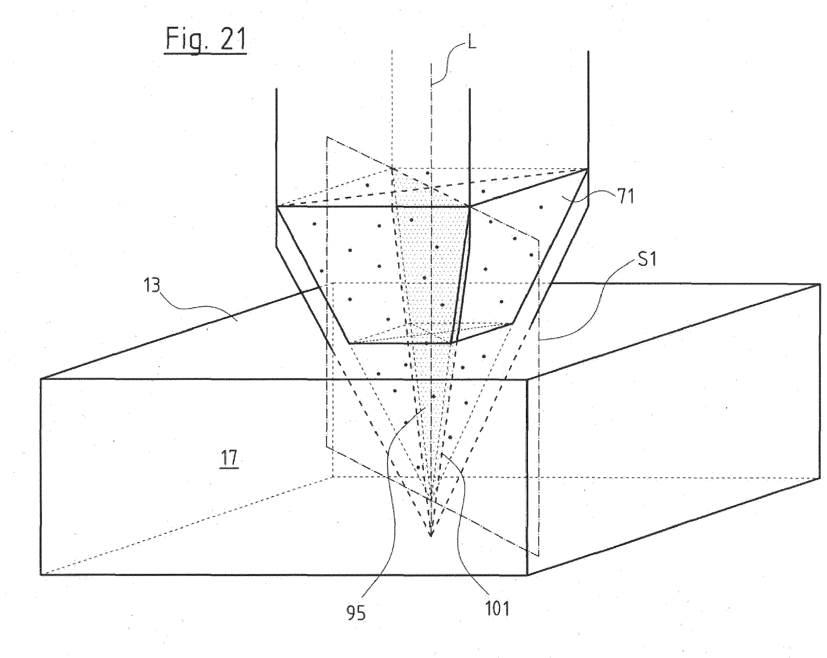

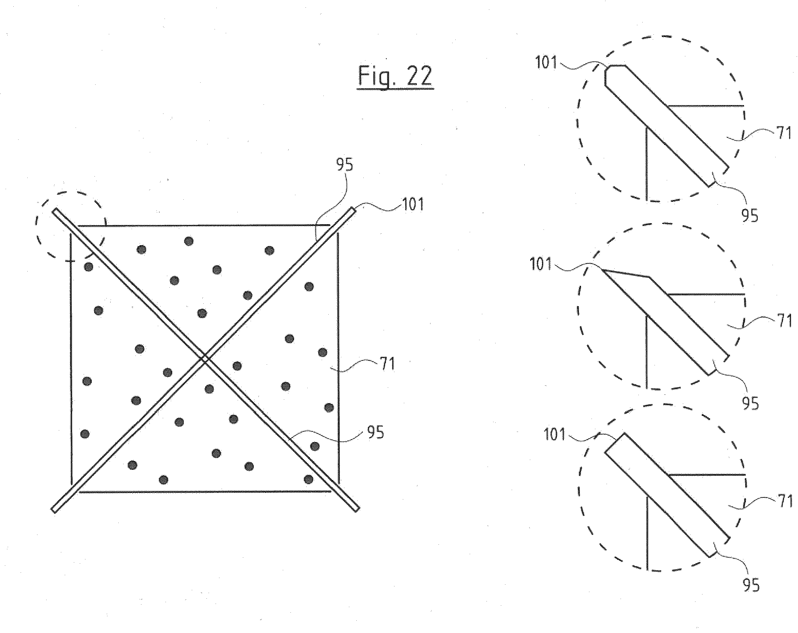

Aus den Figuren 21 und 22 wird deutlich, dass auch bei einem pyramidenförmigen Streukörper 71 mit quadratischer Grundfläche das Verstärkungselement 95 in Form von gekreuzten Klingen 95 zur Bildung von schärferen Schneidkanten 101 über den Streukörper 71 sowohl distal als auch lateral hinausragen kann.

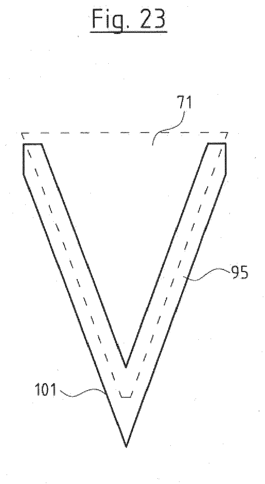

Das in Figur 23 gezeigte Verstärkungselement 95 ist eine V-förmige Klinge, um die von der Klinge 95 im Streukörper 71 gebildete lichtundurchlässige Fläche in der Schneidebene zu verringern. Bei den Klingen gemäß Figuren 17 bis 22 kann die Klingenfläche allerdings gut lichtreflektierend ausgestaltet sein, sodass dies gut kompensiert werden kann.

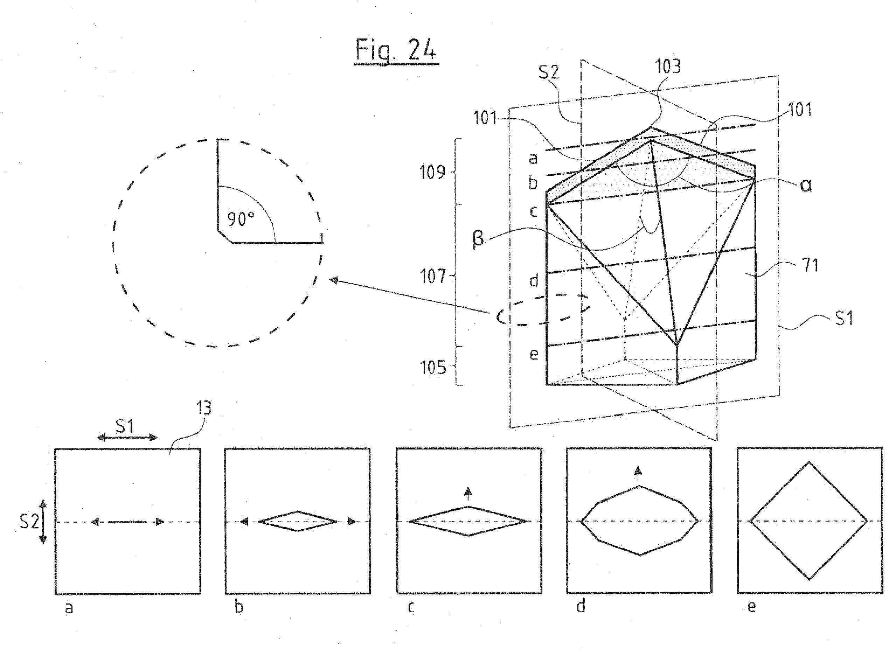

Bei den in Figuren 24 bis 27 gezeigten Ausführungsbeispielen wird bewusst auf eine gekreuzte Konfiguration von zwei Klingen als Verstärkungselement 95 verzichtet und nur eine Klinge als Verstärkungselement 95 eingesetzt. Der Heilungs- und Vernarbungsprozess eines gekreuzten haut- oder Gewebeschnitts kann in manchen Fällen schlechter verlaufen als ein einzelner Längsschnitt. Außerdem ist die Einbettung einer einzelnen Klinge 95 in den Streukörper 71 fertigungstechnisch einfacher.

Der Streukörper 71 hat in den in Figuren 24 bis 27 gezeigten Ausführungsbeispielen eine besondere polyedrische Form mit quadratischer Grundfläche. Der Streukörper 71 läuft in einer ersten Längsschnittebene S1 in einem ersten Winkel α distalwärts spitz zu und in einer zur ersten Längsschnittebene S1 senkrecht liegenden zweiten Längsschnittebene S2 in einem zweiten Winkel β distalwärts spitz zu, wobei der zweite Winkel β spitzer ist als der erste Winkel α. In der ersten Längsschnittebene S1 ist die Klinge 95 als Verstärkungselement eingebettet und ragt distal, aber nicht lateral aus dem Streukörper 71 heraus. Die Schnittkanten 101 der Klinge 95 verlaufen im Winkel α zueinander und bilden eine Spitze 103. In der zweiten Längsschnittebene S2 wird das Gewebe nicht geschnitten, sondern behutsam auseinandergedrückt, und zwar erst dann, wenn der Schnitt in der ersten Längsschnittebene S1 zum Großteil bereits gemacht wurde. Zum behutsamen Auseinanderdrücken des Gewebes in der Längsschnittebene S2 tragen auch die stumpfen Winkel der Kanten des Streukörpers 71 in der Längsschnittebene S2 bei.

In Fig. 24 unten sind die Phasen a-e der Haut- bzw. Gewebeöffnung gezeigt, wenn die Nadelspitze die entsprechend gekennzeichnete Eindringtiefe erreicht hat. Anfänglich in Phase a wird nur in der ersten Längsschnittebene S1 geschnitten und in Phase b nur wenig zusätzlich in der zweiten Längsschnittebene S2 das Gewebe auseinandergedrückt. In Phase c ist der Schnitt in der ersten Längsschnittebene S1 vollendet. Der Großteil der Aufweitung der Haut- bzw. Gewebeöffnung in der zweiten Längsschnittebene S2 findet erst in den Phasen d und e statt, also dann, wenn bereits die Gewebeöffnung durch einen definierten Schnitt erzielt wurde.

Der Streukörper 71 gemäß den in Figuren 24 bis 27 gezeigten Ausführungsbeispielen weist einen ersten proximalen Streukörperabschnitt 105 mit quadratischem Querschnitt auf (siehe Phase e). Distal vom ersten Streukörperabschnitt 105 erstreckt sich ein zweiter Streukörperabschnitt 107 und distal vom zweiten Streukörperabschnitt 107 erstreckt sich ein dritter Streukörperabschnitt 109. Der zweite Streukörperabschnitt 107 hat einen im Wesentlichen achteckigen Querschnitt (siehe Phase d) und der dritte Streukörperabschnitt 109 hat einen im Wesentlichen rautenförmigen Querschnitt (siehe Phasen b und c). Das Verstärkungselement 95 verläuft in der Längsschnittebene S1 entlang der längeren Rautendiagonalen im dritten Streukörperabschnitt 109.

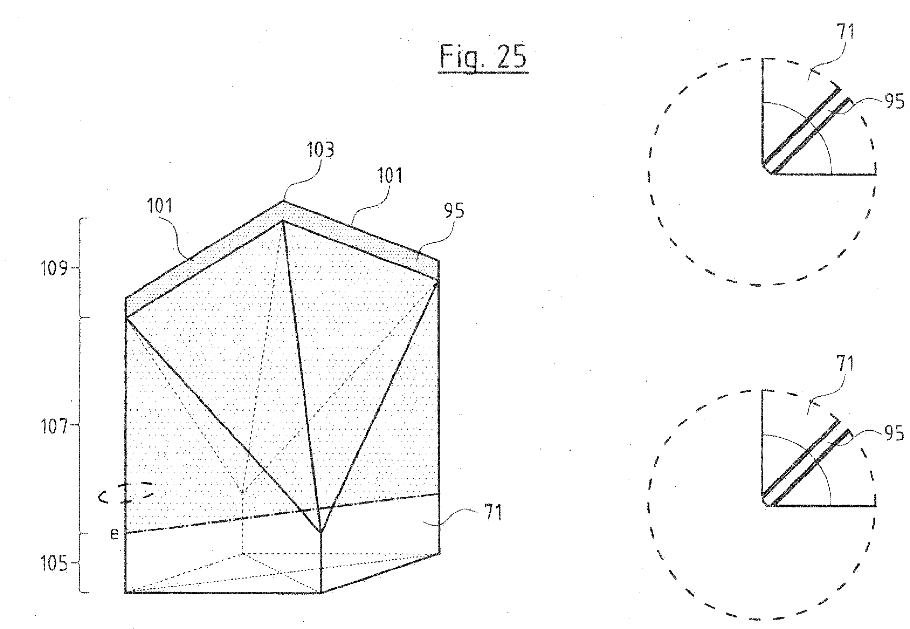

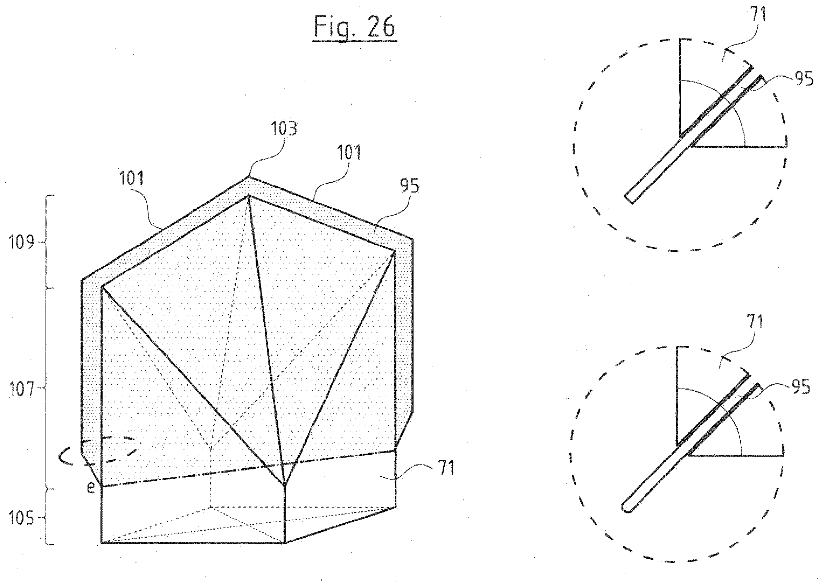

Wie in Fig. 25 und 26 gezeigt, kann sich die Klinge 95 auch über den zweiten Streukörperabschnitt 107 erstrecken und lateral mit dem Streukörper 71 abschließen (Fig. 25) oder lateral herausragen (Fig. 26). Bei festerem Gewebe kann eine größere Klinge 95 (Fig. 26) vorteilhaft sein, bei weicherem Gewebe eine kleinere Klinge 95 (Fig. 25). Die in Fig. 26 gezeigte, lateral über den Streukörper 71 hinausragende Klinge 95 bewirkt einen definierten Gewebeschnitt entlang der Klinge 95. Bevor das Gewebe durch den Prozess des Aufweitens während des Vorschiebens des Applikators an einer undefinierten anderen Stelle aufreißt, soll es gezielt in der Ebene der Klinge 95 aufgeschnitten und damit die Gewebeöffnung vergrößert werden.

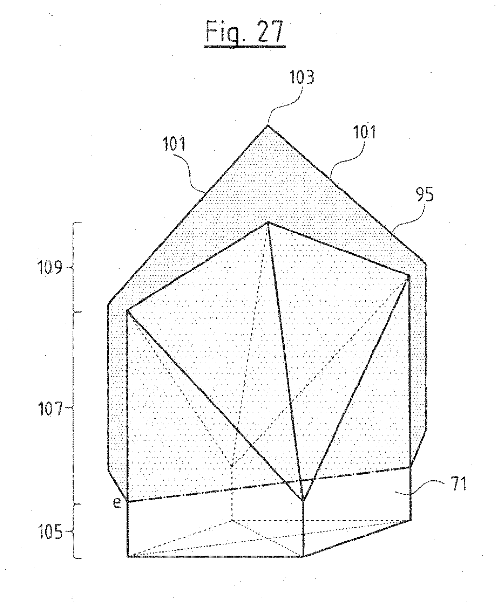

In Fig. 27 ist eine vorteilhafte Ausführungsform gezeigt, bei der die Klinge 95 wie in Fig. 26 lateral herausragt, und dessen Schnittkanten 101 spitzer zueinander laufen als der Winkel α und somit eine spitzere Spitze 103 bilden. Das Verstärkungselement 95 ragt daher mit der Spitze 103 in der radialen Mitte des Streukörpers 71 weiter distalwärts aus dem Streukörper 71 als am radialen Außenbereich des Streukörpers 71. Dies vereinfacht den Einstich in festes Gewebe, wobei u.a. die vom Anwender aufzubringende Kraft reduziert wird.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Im Übrigen soll hierin weder der Begriff "aufweisen" zusätzliche andere Merkmale oder Verfahrensschritte ausschließen noch soll "ein" oder "eine" eine Mehrzahl ausschließen.

Bezugszeichenliste:

| | |
|---|---|
| 1 | Lichtapplikatorsystem |
| 3 | Lichtapplikatorbetriebseinheit |
| 5 | Lichtapplikator |
| 7 | Kabelabschnitt des Lichtapplikators |
| 9 | Stecker des Lichtapplikators |
| 11 | Einführabschnitt des Lichtapplikators |
| 13 | Haut |
| 15 | gesundes Gewebe |
| 17 | pathologisches Gewebe |
| 19 | LED |
| 21 | Anschluss der Lichtapplikatorbetriebseinheit |
| 23 | Versorgungsmodul |
| 25 | Steuerungsmodul |
| 27 | Organ |
| 29 | Flachbandkabel |
| 31 | Arbeitskanal |
| 33 | Schaft |
| 35 | Schaftinstrument |
| 37 | Einführabschnitt des Endoskops |
| 39 | Bildsensor |
| 41 | erster Schaftabschnitt |
| 43 | zweiter Schaftabschnitt |
| 45 | dritter Schaftabschnitt |
| 49, 49a, 49b | Ummantelung des Lichtapplikators |
| 50 | erster elektrischer Kontakt der LED |
| 51 | zweiter elektrischer Kontakt der LED |

| | |
|---|---|
| 53 | Adapterstück |
| 55 | Metallhälfte |
| 57 | Metallhälfte |
| 59 | Isolator-Schicht |
| 61 | elektrischer Leiter |
| 61a | erster elektrischer Leiter |
| 61b | zweiter elektrischer Leiter |
| 63 | Kontaktaufnahme |
| 65 | Kontaktaufnahme |
| 67 | Hülse |
| 69 | Kontaktbügel |
| 71 | Streukörper |
| 73 | Verbindungsanschluss |
| 74 | Kern des ersten elektrischen Leiters |
| 75 | proximaler Nadelspitzenabschnitt bzw. erste Wärmedämmschicht |
| 76 | Mantel des ersten elektrischen Leiters |
| 77 | zweite Wärmedämmschicht |
| 79 | dritte Wärmedämmschicht |
| 80 | Vertiefung |
| 81 | Stirnfläche des Streukörpers |
| 82 | Aufweitung |
| 83 | Kante des Streukörpers |
| 84 | Hinterschnitt |
| 85 | Spitze des Streukörpers |
| 87 | lichtstreuendes Material |
| 89 | von der LED abgestrahltes Licht |
| 91 | vom Streukörper abgestrahltes Licht |
| 93 | Nadelspitzenendbereich des Streukörpers |
| 95 | Verstärkungselement |
| 97 | Kanten des Streukörpers |
| 99 | Schlitze im Verstärkungselement |
| 101 | Schneidkanten des Verstärkungselements |
| 103 | Spitze des Verstärkungselements |
| 105 | erster Streukörperabschnitt |
| 107 | zweiter Streukörperabschnitt |
| 109 | dritter Streukörperabschnitt |
| A | erster Lichtapplikatorabschnitt |
| B | zweiter Lichtapplikatorabschnitt |
| C | dritter Lichtapplikatorabschnitt |
| D | vierter Lichtapplikatorabschnitt |
| L | Längsachse des Lichtapplikators |
| S1 | erste Längsschnittebene |
| S1 | zweite Längsschnittebene |
| α | erster Winkel |
| β | zweiter Winkel |

**Patentansprüche**

**1.** Lichtapplikator (5) zur Untersuchung und/oder Behandlung von einem organischen Körper, wobei der Lichtapplikator (5) einen distalseitigen Nadelabschnitt (11) mit einer LED (19) am distalen Ende zum Einstechen in Gewebe (17) des organischen Körpers und einen proximalseitigen Kabelabschnitt (7) aufweist,

wobei der Lichtapplikator (5) einen elektrischen Leiter (61) zur Stromversorgung der LED (19) aufweist,
**dadurch gekennzeichnet, dass**
der Leiter (61) im Nadelabschnitt (11) massiv und in einer Weise dicker als im Kabelabschnitt (7) ausgeführt ist, dass die Querschnittsfläche des Leiters (61) im Nadelabschnitt mehr als 70% der Querschnittsfläche des Lichtapplikators (5) beträgt und somit der Nadelabschnitt (11) biegesteifer als der Kabelabschnitt (7) ist.

2. Lichtapplikator (5) nach einem der vorhergehenden Ansprüche, wobei der Nadelabschnitt (11) und der Kabelabschnitt (7) lösbar miteinander verbindbar sind.

3. Lichtapplikator (5) nach einem der vorhergehenden Ansprüche, wobei der Leiter (61) einen Kern (74) mit einem ersten Material und einen Mantel (76) mit einem zweiten Material aufweist, wobei das erste Material wärmeleitfähiger ist als das zweite Material und das zweite Material biegesteifer ist als das erste Material.

4. Lichtapplikator (5) nach Anspruch 3, wobei der Kern (74) als Hinleiter und der Mantel (76) als Rückleiter genutzt wird oder umgekehrt.

5. Lichtapplikator (5) nach Anspruch 3 oder 4, wobei der Kern (74) und der Mantel (76) durch eine dünne, elektrisch nichtleitende Schicht, die zwischen dem Kern (74) und dem Mantel (76) angeordnet ist, gegeneinander elektrisch isoliert sind.

6. Lichtapplikator (5) nach einem der Ansprüche 3 bis 5, wobei das erste Material Kupfer, Aluminium oder Silber und das zweite Material Stahl aufweist.

7. Lichtapplikator (5) nach einem der Ansprüche 3 bis 6, wobei die Querschnittsfläche des Kerns (74) mehr als 40% der Querschnittsfläche des Leiters (61) beträgt.

8. Lichtapplikator (5) nach einem der Ansprüche 3 bis 7, wobei die Querschnittsfläche des Kerns das 0,8 bis 1,2-fache der Querschnittsfläche der LED beträgt.

9. Lichtapplikator (5) nach einem der vorhergehenden Ansprüche, wobei die Querschnittsfläche des Leiters (61) mindestens so groß wie die Querschnittsfläche der LED (19) ist.

10. Lichtapplikator (5) nach einem der vorhergehenden Ansprüche, wobei der Leiter (61) außenseitig mindestens eine Vertiefung (80) und/oder Aufweitung (82) aufweist.

11. Lichtapplikator (5) nach einem der vorhergehenden Ansprüche, wobei die LED (19) in elektrischem und wärmeleitendem Kontakt mit einer distalen Stirnfläche des Leiters (61) steht.

12. Lichtapplikator (5) nach einem der vorhergehenden Ansprüche, wobei der Lichtapplikator (5) im Nadelabschnitt (11) in radiale Richtung derart wärmegedämmt ist, dass die radiale Wärmedämmung proximalwärts abnimmt.

13. Lichtapplikator (5) nach Anspruch 12, wobei die radiale Wärmedämmung im Nadelabschnitt (11) proximalwärts stufenweise und/oder stetig abnimmt.

14. Lichtapplikator (5) nach Anspruch 12 oder 13, wobei der Lichtapplikator (5) im Nadelabschnitt (11) mindestens eine radiale Wärmedämmschicht (75, 77, 79) aufweist, wobei die Gesamtdicke der mindestens einen Wärmedämmschicht (75, 77, 79) proximalwärts abnimmt.

15. Lichtapplikator (5) nach einem der Ansprüche 12 bis 14, wobei der Lichtapplikator (5) im Nadelabschnitt (11) eine Mehrzahl von radialen Wärmedämmschichten (75, 77, 79) aufweist, wobei die Anzahl der Wärmedämmschichten (75, 77, 79) proximalwärts abnimmt.

**Claims**

1. Light applicator (5) for the examination and/or treatment of an organic body, wherein

the light applicator (5) comprises a distal side needle section (11) with an LED (19) at the distal end for purposes of piercing the tissue (17) of the organic body, and a proximal-side cable section (7), wherein
the light applicator (5) comprises an electrical conductor (61) for the supply of power to the LED (19),
**characterised in that**,
the conductor (61) in the needle section (11) is embodied so as to be solid, and thicker than in the cable section (7), such that the cross-sectional area of the conductor (61) in the needle section is more than 70% of the cross-sectional area of the light applicator (5), and thus the needle section (11) is more resistant to bending than the cable section (7).

2. Light applicator (5) according to one of the preceding claims, wherein the needle section (11) and the cable section (7) can be connected to each other in a detachable manner.

3. Light applicator (5) according to one of the preceding claims, wherein the conductor (61) comprises a core (74) with a first material, and a jacket (76) with a second material, wherein the first material is more thermally conductive than the second material, and the second material is more resistant to bending than the first material.

4. Light applicator (5) according to Claim 3, wherein the core (74) is used as a forward conductor, and the jacket (76) is used as a return conductor, or vice versa.

5. Light applicator (5) according to Claim 3 or 4, wherein the core (74) and the jacket (76) are electrically insulated from each other by a thin, electrically nonconductive layer, which is arranged between the core (74) and the jacket (76).

6. Light applicator (5) according to one of the Claims 3 to 5, wherein the first material comprises copper, aluminium, or silver, and the second material comprises steel.

7. Light applicator (5) according to one of the Claims 3 to 6, wherein the cross-sectional area of the core (74) is more than 40% of the cross-sectional area of the conductor (61).

8. Light applicator (5) according to one of the Claims 3 to 7, wherein the cross-sectional area of the core is 0.8 to 1.2 times the cross-sectional area of the LED.

9. Light applicator (5) according to one of the preceding claims, wherein the cross-sectional area of the conductor (61) is at least as large as the cross-sectional area of the LED (19).

10. Light applicator (5) according to one of the preceding claims, wherein the conductor (61) comprises at least one depression (80) and/or widening (82) on the outside.

11. Light applicator (5) according to one of the preceding claims, wherein the LED (19) stands in electrical and thermal contact with a distal end face of the conductor (61).

12. Light applicator (5) according to one of the preceding claims, wherein the light applicator (5) in the needle section (11) is thermally insulated in the radial direction, such that the radial thermal insulation decreases in the proximal direction.

13. Light applicator (5) according to Claim 12, wherein the radial thermal insulation in the needle section (11) decreases in a stepwise manner and/or continuously in the proximal direction.

14. Light applicator (5) according to Claim 12 or 13, wherein the light applicator (5) has at least one radial thermal barrier layer (75, 77, 79) in the needle section (11), wherein the total thickness of the at least one thermal barrier layer (75, 77, 79) decreases in the proximal direction.

15. Light applicator (5) according to one of the Claims 12 to 14, wherein the light applicator (5) has a plurality of radial thermal barrier layers (75, 77, 79) in the needle section (11), wherein the number of thermal barrier layers (75, 77, 79) decreases in the proximal direction.

**Revendications**

1. Applicateur de lumière (5) pour l'examen et/ou le traitement d'un corps organique, l'applicateur de lumière (5) comprenant, du côté distal, une partie d'aiguille (11) avec une DEL (19) à l'extrémité distale, destinée à être introduite dans un tissu (17) du corps organique, et une partie de câble (7) du côté proximal, l'applicateur de lumière (5) comprenant un conducteur d'électricité (61) pour l'alimentation en électricité de la DEL (19),
   **caractérisé en ce que**
   dans la partie d'aiguille (11), le conducteur (61) est réalisé de façon massive et est réalisé de façon plus épaisse que dans la partie de câble (7) afin que, dans la partie d'aiguille, la surface de section transversale du conducteur (61) soit de 70% de la surface de section transversale de l'applicateur de lumière (5) et que, ainsi, la partie d'aiguille (11) soit plus résistante à la flexion que la partie de câble (7).

2. Applicateur de lumière (5) selon l'une des revendications précédentes, la partie d'aiguille (11) et la partie de câble (7) pouvant être reliées l'une à l'autre de façon amovible.

3. Applicateur de lumière (5) selon l'une des revendications précédentes, le conducteur (61) comprenant un noyau (74) avec un premier matériau et une enveloppe (76) avec un deuxième matériau, le premier matériau conduisant mieux la chaleur que le deuxième matériau et le deuxième matériau étant plus résistant à la flexion que le premier matériau.

4. Applicateur de lumière (5) selon la revendication 3, le noyau (74) étant utilisé comme conducteur d'aller et l'enveloppe (76) comme conducteur de retour ou inversement.

**5.** Applicateur de lumière (5) selon la revendication 3 ou 4, le noyau (74) et l'enveloppe (76) étant isolés électriquement l'un par rapport à l'autre par une couche fine électriquement non conductrice qui est disposée entre le noyau (74) et l'enveloppe (76).

**6.** Applicateur de lumière (5) selon l'une des revendications 3 à 5, le premier matériau comprenant du cuivre, de l'aluminium ou de l'argent et le deuxième matériau comprenant de l'acier.

**7.** Applicateur de lumière (5) selon l'une des revendications 3 à 6, la surface de la section transversale du noyau (74) étant supérieure à 40% de la surface de la section transversale du conducteur (61).

**8.** Applicateur de lumière (5) selon l'une des revendications 3 à 7, la surface de la section transversale du noyau étant 0,8 à 1,2 fois la surface de la section transversale de la DEL.

**9.** Applicateur de lumière (5) selon l'une des revendications précédentes, la surface de la section transversale du conducteur (61) étant au moins aussi grande que la surface de la section transversale de la DEL (19).

**10.** Applicateur de lumière (5) selon l'une des revendications précédentes, le conducteur (61) comprenant sur le côté extérieur au moins un renfoncement (80) et/ou un élargissement (82).

**11.** Applicateur de lumière (5) selon l'une des revendications précédentes, la DEL (19) étant en contact électrique et thermoconducteur avec une surface frontale (61) distale du conducteur (61).

**12.** Applicateur de lumière (5) selon l'une des revendications précédentes, dans la partie d'aiguille (11), l'applicateur de lumière (5) étant isolé thermiquement en direction radiale de manière telle que l'isolation thermique radiale diminue en direction proximale.

**13.** Applicateur de lumière (5) selon la revendication 12, l'isolation thermique radiale dans la partie d'aiguille (11) diminuant en direction proximale par étapes et/ou de manière constante.

**14.** Applicateur de lumière (5) selon la revendication 12 ou 13, dans la partie d'aiguille (11), l'applicateur de lumière (5) comprenant au moins une couche d'isolation thermique radiale (75, 77, 79), l'épaisseur totale de ladite au moins une couche d'isolation thermique (75, 77, 79) diminuant en direction proximale.

**15.** Applicateur de lumière (5) selon l'une des revendications 12 à 14, dans la partie d'aiguille (11), l'applicateur de lumière (5) comprenant une quantité de couches d'isolation thermique radiales (75, 77, 79), le nombre de couches d'isolation thermique (75, 77, 79) diminuant en direction proximale.

Fig. 1

Fig. 2

EP 3 777 974 B1

Fig. 3

# Fig. 4

EP 3 777 974 B1

EP 3 777 974 B1

Fig. 5

Fig. 6

Fig. 7

EP 3 777 974 B1

## Fig. 8

EP 3 777 974 B1

Fig. 9a

Fig. 9b

61a

61a

74

76

19

19

74

76

19

a

Fig. 10a

Fig. 10b

61a

61a

74

76

19

19

74

76

19

a

EP 3 777 974 B1

28

## Fig. 11a

## Fig. 11b

Fig. 11c

Fig. 11d

EP 3 777 974 B1

Fig. 12a  Fig. 12b  Fig. 12c

Fig. 13

## Fig. 14

## Fig. 15

EP 3 777 974 B1

Fig. 16

Fig. 17

Fig. 18

Fig. 19

95  71  95  97

Fig. 20

EP 3 777 974 B1

EP 3 777 974 B1

Fig. 21

Fig. 22

Fig. 23

EP 3 777 974 B1

Fig. 24

Fig. 25

EP 3 777 974 B1

Fig. 26

EP 3 777 974 B1

Fig. 27

**EP 3 777 974 B1**

**IN DER BESCHREIBUNG AUFGEFÜHRTE DOKUMENTE**

**In der Beschreibung aufgeführte Patentdokumente**

- EP 2449994 A1
- US 2016151639 A1
- US 2016213945 A1
- US 20140188035 A1